(19) Europäisches Patentamt
European Patent Office
Office européen des brevets

(11) EP 1 854 893 A1

(12) # EUROPEAN PATENT APPLICATION
published in accordance with Art. 158(3) EPC

(43) Date of publication:
**14.11.2007 Bulletin 2007/46**

(21) Application number: **06714508.6**

(22) Date of filing: **24.02.2006**

(51) Int Cl.:
***C12P 41/00*** *(2006.01)* ***C12N 15/53*** *(2006.01)*

(86) International application number:
**PCT/JP2006/303369**

(87) International publication number:
**WO 2006/090814 (31.08.2006 Gazette 2006/35)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **25.02.2005 JP 2005050488**

(71) Applicant: **Kaneka Corporation**
**Kita-ku**
**Osaka-shi, Osaka 530-8288 (JP)**

(72) Inventors:
- **IWASAKI, Akira**
**, 6750068 (JP)**
- **WASHIDA, Motohisa**
**Hyog 655-0893 (JP)**
- **TAOKA, Naoaki**
**6540122 (JP)**
- **MORIYAMA, Daisuke**
**go, 6550872 (JP)**
- **HASEGAWA, Junzou**
**o, 6740057 (JP)**

(74) Representative: **HOFFMANN EITLE**
**Patent- und Rechtsanwälte**
**Arabellastrasse 4**
**81925 München (DE)**

(54) ## PROCESS FOR PRODUCING OPTICALLY ACTIVE SECONDARY ALCOHOL

(57) The present invention provides a method enabling convenient production of an optically active secondary alcohol useful as a pharmaceutical intermediate, particularly an optically active 1,2-diol and an optically active 2-alkanol, from an enantiomer mixture thereof.

An oxidizing enzyme source having the capability of selectively oxidizing one enantiomer of secondary alcohol is allowed to act on an enantiomer mixture of secondary alcohol in the presence of a reducing enzyme source having the capability of reverse enantio-selectively reducing a ketone derivative, to convert the enantiomer mixture into a substantially single enantiomer at a theoretical percent recovery of 100%, whereby an optically active secondary alcohol is produced.

EP 1 854 893 A1

## Description

### Technical Field

The present invention relates to a method of producing an optically active secondary alcohol, including an optically active diol, useful as a pharmaceutical intermediate, from an enantiomer mixture thereof.

### Background Art

Conventionally, as methods of producing an optically active secondary alcohol, including an optically active diol, from an enantiomer mixture thereof, the methods described below are known.

1) A method comprising degrading one isomer of racemic 3-chloro-1,2-propanediol using a microorganism to obtain an residual optically active 3-chloro-1,2-propanediol. (Patent document 1).
2) A method comprising allowing cells of *Candida parapsilosis* to act on 1,2-pentanediol racemate, to produce (S)-1,2-pentanediol at a percent recovery exceeding 50% (non-patent documents 1 and 2).
3) The S form of racemic 1, 3-butanediol is oxidized to 4-hydroxy-2-butanone using an *Escherichia coli* that produces the secondary alcohol dehydrogenase derived from *Candida parapsilosis,* and then the cells of the microorganism are removed. Thereafter, an *Escherichia coli* that produces the (R)-2,3-butanediol dehydrogenase derived from *Kluyveromyces lactis* is added to the above-described reaction product to reduce the 4-hydroxy-2-butanone in the reaction product to the R form, whereby (R)-1,3-butanediol is produced (patent document 2).

However, in the method 1), the percent recovery is up to 50%. Additionally, the choice of alcohols to which it is applicable is limited. In the method 2), because the percent recovery exceeds 50%, it is postulated that enantiomer reversal has occurred. However, in non-patent document 1, no details of the oxidizing enzyme and reducing enzyme involved in the reaction are clarified, and in non-patent document 2, some of the enzymes involved in the reaction are deduced; however, because both methods comprise oxidizing an alcohol to a ketone and reducing the ketone to an alcohol having the reverse configuration in cells of a single microorganism, a combination of an oxidizing enzyme and a reducing enzyme is fixed, so that the choice of alcohols to which this method is applicable is limited. Furthermore, because the reaction cannot be efficiently performed using the oxidative reaction and/or the reductive reaction in combination with a coenzyme regeneration system, the productivity is insufficient. In the method 3), the theoretical percent recovery is 100%, but because it is necessary to remove the cells utilized for the oxidative reaction before performing the reductive reaction, the steps are complicated. Furthermore, because a ketone compound is once accumulated, a) the oxidative reaction undergoes product inhibition, b) the reductive reaction undergoes substrate inhibition, c) the oxidizing enzyme and/or the reducing enzyme are inactivated, d) if the ketone is unstable, percent yield is reduced due to degradation of the ketone, and other problems arise.
As stated above, all these methods have problems to be solved for industrial processes, including low percent recovery, low productivity, step complexity and the like.

Patent document 1: JP-A-63-251098
Patent document 2: JP-A-2002-345479
Non-patent document 1: Agric. Biol. Chem., 54(7), 1819-1827, 1990
Non-patent document 2: Organic Process Research & Development, 8 (2), 246-251 (2004)

### Disclosure of the Invention

### Problems to be solved by the invention

In view of the above-described circumstances, it is an object of the present invention to provide a method of producing an optically active alcohol useful as an intermediate for a pharmaceutical from an enantiomer mixture thereof conveniently and at high percent recovery.

### Means of solving the problems

The present inventors conducted diligent investigations of the above-described problems and, as a result, found a method comprising converting an enantiomer mixture of secondary alcohol to a substantially single enantiomer at a theoretical percent recovery of 100% and conveniently by using in combination an oxidizing enzyme source and reducing enzyme source having particular properties, and developed the present invention.

An outline of the present invention is given in the following scheme.

Scheme 1

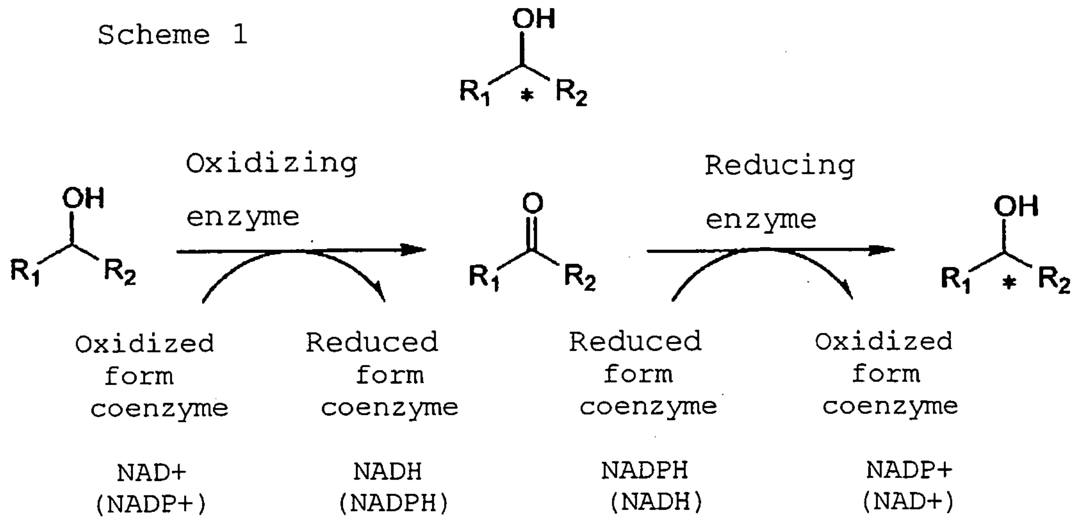


The present invention is a method of producing an optically active alcohol by, as shown in scheme 1, converting an enantiomer mixture of secondary alcohol to a substantially single enantiomer at a theoretical percent recovery of 100%, that is, deracemizing the mixture, in the co-presence of an oxidizing enzyme source having the capability of selectively oxidizing one enantiomer of the enantiomer mixture of secondary alcohol and a reducing enzyme source having the reverse enantio-selectivity to that of the oxidizing enzyme source, and having the capability of reducing the ketone compound resulting from the oxidative reaction to an optically active secondary alcohol.

Although there is a known method comprising performing an oxidative reaction and a reductive reaction in two separate stages as in the foregoing patent document 2, the method of the present invention, unlike it, is characterized in that the reactions are performed in the co-presence of an oxidizing enzyme source and a reducing enzyme source, that is, the oxidative reaction and the reductive reaction are performed simultaneously.

Here, it should be noted that the deracemation of the present invention cannot be achieved simply by allowing an oxidizing enzyme source and a reducing enzyme source to be co-present. As the enzyme acting in the above-described oxidative or reductive reaction in the present invention (oxidizing enzyme source, reducing enzyme source), oxidoreductases such as dehydrogenase, classified under E.C.1.1.1, are used; these enzymes require coenzymes such as nicotinamide adenine dinucleotide (NAD+ (NADH)) and nicotinamide adenine dinucleotide phosphate (NADP+ (NADPH)), and generally reversibly catalyze the reactions (catalyze both the oxidative reaction and the reductive reaction). Therefore, to efficiently perform deracemation, it is necessary to establish conditions that cause the reactions to proceed steadily from the left to the right in the foregoing scheme 1

Usually, when the oxidative reaction is performed using these dehydrogenases, it is desirable that regarding the coenzymes used, the oxidized form (NAD+ or NADP+) be present in excess to the reduced form (NADH or NADPH); on the other hand, when the reductive reaction is performed, it is desirable that regarding the coenzymes, the reduced form be present in excess to the oxidized form. However, if the coenzyme specificities for the oxidizing enzyme and the reducing enzyme are the same in the deracemation reaction shown by scheme 1, the oxidized form and the reduced form of coenzymes that can be used by both enzymes occur in considerable amounts in the reaction system; therefore, the reverse reactions to the desired reactions (oxidative and reductive reactions) also proceed simultaneously; as a result, the deracemation reaction does not proceed efficiently.

Hence, the present inventors diligently investigated with the aim of solving the above-described problems and, as a result, found that by using in combination an oxidizing enzyme source and a reducing enzyme source having different specificities for coenzymes, the deracemation reaction proceeded efficiently, and developed the present invention. Here, "having different specificities for coenzymes" means that if the oxidizing enzyme source is specific for NAD+, the reducing enzyme source is specific for NADPH, or that if the oxidizing enzyme source is specific for NADP+, the reducing enzyme source is specific for NADH.

In other words, the present invention relates to a method of producing an optically active secondary alcohol by converting an enantiomer mixture of secondary alcohol into an optically active secondary alcohol consisting of a substantially single enantiomer, comprising performing the converting reaction in the co-presence of an oxidizing enzyme source having the following property (1) and a reducing enzyme source having the following property (2):

(1) the oxidizing enzyme source exhibits specificity for one of the oxidized form coenzymes NAD+ or NADP+, and has an activity to selectively oxidize one enantiomer of the S form or R form of secondary alcohol to produce a corresponding ketone compound,
(2) the reducing enzyme source exhibits specificity for one of the reduced form coenzymes NADPH and NADH (here, if the oxidizing enzyme source is specific for NAD+, the reducing enzyme source is specific for NADPH; if the oxidizing enzyme source is specific for NADP+, the reducing enzyme source is specific for NADH), has the reverse enantio-selectivity to that of the oxidizing enzyme source, and has an activity to reduce the foregoing ketone compound to produce the S form (or R form) of secondary alcohol.

Thus, in the production method of the present invention, the oxidative reaction and the reductive reaction can be simultaneously performed, and there is no need for removing the oxidizing enzyme before starting the reductive reaction, so that the steps are simplified. Because the resulting ketone compound is rapidly reduced, an optically active secondary alcohol can be produced extremely efficiently, while avoiding the above-described various problems due to ketone accumulation.

**Effect of the invention**

According to the method of the present invention, an optically active alcohol, including an optically active diol, useful as a pharmaceutical intermediate, can be produced conveniently at high yields from an enantiomer mixture thereof, particularly from an inexpensive racemate.

**Brief Description of the Drawings**

[Figure 1] An illustration showing how to prepare the recombinant vector pNTNX.
[Figure 2] A restriction enzyme map of the recombinant vector pTSOB incorporating the dehydrogenase gene derived from *Ochrobactrum* sp. KNKc71-3.

**Best Mode for Embodying the Invention**

The present invention is hereinafter described in detail.
As mentioned above, the present invention relates to a method of producing an optically active secondary alcohol by converting an enantiomer mixture of secondary alcohol into an optically active secondary alcohol consisting of a substantially single enantiomer, comprising performing the converting reaction in the co-presence of an oxidizing enzyme source having the following property (1) and a reducing enzyme source having the following property (2):

(1) the oxidizing enzyme source exhibits specificity for one of the oxidized form coenzymes NAD+ or NADP+, and has an activity to selectively oxidize one enantiomer of the S form or R form of secondary alcohol to produce a corresponding ketone compound,
(2) the reducing enzyme source exhibits specificity for one of the reduced form coenzymes NADPH and NADH (here, if the oxidizing enzyme source is specific for NAD+, the reducing enzyme source is specific for NADPH; if the oxidizing enzyme source is specific for NADP+, the reducing enzyme source is specific for NADH), has the reverse enantio-selectivity to that of the oxidizing enzyme source, and has an activity to reduce the foregoing ketone compound to produce the S form (or R form) of secondary alcohol.

The gist of the present invention resides in a reaction system (deracemization reaction system) capable of producing an optically active secondary alcohol consisting of a substantially single enantiomer, irrespective of the type of secondary alcohol, from an enantiomer mixture thereof, at a theoretical percent recovery of 100%. Therefore, the present invention is not limited by any means by the choice of secondary alcohol produced, and by the derivation and form of the enzyme sources used for the oxidative and reductive reactions.
In the present invention, "an enantiomer mixture of secondary alcohol" generically means all secondary alcohols whose optical purity does not meet the requirement for the intended use. In addition to racemates, for example, if an (R) form of 90% e.e. is demanded, secondary alcohols having an (R) form content of less than 90% e.e., including (S) forms of high optical purity, are all encompassed in the scope of the present invention. Usually, because racemates are inexpensive and easily available, the effect of the present invention is maximized when a racemate is used.
In the present invention, "an optically active secondary alcohol consisting of a substantially single enantiomer" means a secondary alcohol whose optical purity meets the requirement for the intended use, and does not always need

to have an optical purity of 100% e.e. For example, for a pharmaceutical intermediate, the optical purity of the desired enantiomer is not less than 90% e.e., preferably not less than 95% e.e., more preferably not less than 98% e.e., and of course the requirement varies depending on factors such as optical purity improvability in the subsequent steps.

In the present invention, "the reducing enzyme source has the reverse enantio-selectivity to that of the oxidizing enzyme source" means that if the oxidizing enzyme source selectively oxidizes the R form of a secondary alcohol to produce a ketone compound, the reducing enzyme source reduces the ketone compound to selectively produce the S form of the secondary alcohol, or that if the oxidizing enzyme source selectively oxidizes the S form of a secondary alcohol to produce a ketone compound, the reducing enzyme source reduces the ketone compound to selectively produce the R form of the secondary alcohol.

The enzymes acting for the enantio-selective oxidative reaction and reductive reaction in the present invention are, as described above, oxidoreductases such as dehydrogenase, classified under E.C.1.1.1. In the present invention, these enzymes are called "oxidizing enzyme (oxidizing enzyme source)" when used in the oxidative reaction, and "reducing enzyme (reducing enzyme source)" when used in the reductive reaction.

In the present invention, "the oxidizing enzyme is specific for NAD+ (or NADP+)" means that if NAD+ (or NADP+) is used as the coenzyme, higher activity is exhibited (specificity is exhibited) than if the other is used as the coenzyme. The ratio of the activity with the use of the coenzyme exhibiting specificity and the activity with the use of the other coenzyme is normally not less than 100/50, preferably not less than 100/10, more preferably not less than 100/2.

In the present invention, "the reducing enzyme is specific for NADPH (or NADH)" has the same meaning as that described above, and the ratio of the activity when using the coenzyme that exhibits specificity and the activity when using the other coenzyme is also the same as that described above.

The deracemization reaction of the present invention can be performed by adding the substrate enantiomer mixture of secondary alcohol, nicotinamide adenine dinucleotide (NAD+ (NADH)), nicotinamide adenine dinucleotide phosphate (NADP+ (NADPH)), and the above-described oxidizing enzyme source and reducing enzyme source to an appropriate solvent, and stirring while adjusting the pH; it is preferable that the reaction be performed using coenzyme regeneration systems in combination.

Usually, the oxidative reaction and the reductive reaction using dehydrogenase require stoichiometric amounts of an oxidized form coenzyme and a reduced form coenzyme, respectively; by using in combination an oxidized form coenzyme NAD+ (or NADP+) regeneration system and/or a reduced form coenzyme NADPH (or NADH) regeneration system, the amounts of expensive coenzymes used can be remarkably reduced. Therefore, it is preferable to combine the oxidative reaction and/or reductive reaction that constitutes the above-described deracemation reaction with a coenzyme regeneration system; it is more preferable to combine both the oxidative reaction and the reductive reaction with respective coenzyme regeneration systems.

As a coenzyme regeneration system, first, a method utilizing the coenzyme regeneration capability in a microbial cell, for example, a microbial cell that produces the above-described oxidizing enzyme can be mentioned. When the coenzyme regeneration capability in a microbial cell, for example, a recombinant *Escherichia coli* cell that produces oxidizing enzyme, is utilized to regenerate an oxidized form coenzyme, the oxidative reaction of alcohol and the oxidized form coenzyme regeneration system are separated from outside by the cell membrane; therefore, even if the coenzyme specificity of the enzyme of the reduced form coenzyme regeneration system is low or absent, there are advantages such as the unlikelihood of the above-described failure due to the conjugation of the oxidized form coenzyme regeneration system and the reduced form coenzyme regeneration system.

As another coenzyme regeneration system, a method utilizing an enzyme for regenerating an oxidized form coenzyme and/or a reduced form coenzyme, other than the oxidizing enzyme and reducing enzyme for the deracemation reaction, can be mentioned. In this case, if the reaction for regenerating an oxidized form coenzyme and the reaction for regenerating a reduced form coenzyme are conjugated via the coenzymes as the intermediates, the regeneration of the desired coenzymes does not proceed efficiently. Therefore, it is preferable that the enzymes used for the coenzyme regeneration systems exhibit high specificity for the respective coenzymes. That is, it is preferable that if the oxidizing enzyme is NAD+ specific, the enzyme for the oxidized form coenzyme regeneration system be NADH specific, and the enzyme for the reduced form coenzyme regeneration system be NADP+ specific; likewise, it is preferable that if the oxidizing enzyme is NADP+ specific, the enzyme for the oxidized form coenzyme regeneration system be NADPH-specific, and the enzyme for the reduced form coenzyme regeneration system be NAD+-specific.

As the enzyme having the capability of regenerating an oxidized form coenzyme, NADH oxidase, NADPH dehydrogenase, and amino acid dehydrogenase can be mentioned; among them, NADH oxidase is preferable because of its features such as the availability of oxygen as the substrate for the coenzyme regenerative reaction, the specificity of many of them for NADH, and the irreversibility of the reaction catalyzed. Two types of NADH oxidase are known: one producing water (water-producing NADH oxidase) and one producing hydrogen peroxide (hydrogen peroxide-producing NADH oxidase). Hydrogen peroxide is known to have an adverse effect on enzymes and the like; when hydrogen peroxide-producing NADH oxidase is used, it is preferable to add catalase to the reaction system to thereby decompose hydrogen peroxide and reduce or eliminate the adverse effect thereof. Because the production of hydrogen peroxide

per se is avoided, it is more preferable to use water-producing NADH oxidase.

Although the organism that serves as the source of the above-described water-producing NADH oxidase is not subject to limitation, and may be a microorganism or a higher organism, and microorganisms such as bacteria, fungi, and yeast are suitable; preferably bacteria can be mentioned. For example, a microorganism belonging to the genus *Streptococcus,* the genus *Lactobacillus,* the genus *Lactococcus,* the genus *Leuconostock,* the genus *Entrococcus,* the genus *Pediococcus,* the genus *Methanococcus,* the genus *Serpulina,* the genus *Mycoplasma, or* the genus *Giardia* can be mentioned; preferably a microorganism of the genus *Streptococcus,* more preferably *Streptococcus mutans,* particularly preferably *Streptococcus mutans* NCIB11723, can be mentioned. Regarding the water-producing NADH oxidase derived from *Streptococcus mutans* NCIB11723, the amino acid sequence thereof and the base sequence of the DNA that encodes the same have already been reported (JP-A-8-196281).

As the enzyme having the capability of regenerating a reduced form coenzyme, glucose dehydrogenase, formic acid dehydrogenase, and glucose 6-phosphate dehydrogenase can be mentioned; preferably glucose dehydrogenase, more preferably NADP+-specific glucose dehydrogenase, can be mentioned.

Although the organism that serves as the source of the above-described glucose dehydrogenase is not subject to limitation, and may be a microorganism or a higher organism, and microorganisms such as bacteria, fungi, and yeast are suitable; preferably a bacterium can be mentioned. For example, a microorganism of the genus *Bacillus,* preferably *Bacillus megaterium,* can be mentioned.

Although the organism that serves as the source of the above-described NADP+-specific glucose dehydrogenase is not subject to limitation, and may be a microorganism or a higher organism, and microorganisms such as bacteria, fungi, and yeast are suitable. For example, a microorganism belonging to the genus *Cryptococcus,* the genus *Gluconobacter,* or the genus *Saccharomyces* can be mentioned. Preferably, a microorganism belonging to the genus *Cryptococcus* can be mentioned. As the microorganism of the genus *Cryptococcus, Cryptococcus albi dus, Cryptococcus humicolus, Cryptococus terreus,* and *Cryptococcus uniguttulatus* can be mentioned; preferably *Cryptococcus uniguttulatus,* more preferably the *Cryptococcus uniguttulatus* JCM3687 strain, can be mentioned.

The *Cryptococcus uniguttulatus* JCM3687 strain is stored at the RIKEN Japan Collection of Microorganisms (JMC: 2-1, Hirosawa, Wako-shi, Saitama, 351-0198 Japan), and can be obtained from the facility.

Provided that the same reaction as described above is performed using a culture product of a recombinant microorganism prepared by introducing an oxidizing enzyme gene and a gene for an enzyme having the capability of regenerating the oxidized form coenzyme on which this enzyme depends (for example, NADH oxidase) into cells of the same host, or a treatment product of the culture product and the like, it is unnecessary to separately prepare the enzyme source necessary for coenzyme regeneration; therefore, an optically active alcohol can be produced at lower costs. Likewise, provided that the same reaction as described above is performed using a culture product of a recombinant microorganism prepared by introducing a reducing enzyme gene and a gene for an enzyme having the capability of regenerating the reduced form coenzyme on which this enzyme depends (for example, glucose dehydrogenase) into cells of the same host, or a treatment product of the culture product and the like, it is unnecessary to separately prepare the enzyme source necessary for coenzyme regeneration; therefore, an optically active diol can be produced at lower costs.

Provided that for the oxidative reaction system (alcohol oxidation and oxidized form coenzyme regeneration system) or the reductive reaction system (ketone derivative reductive reaction and reduced form coenzyme regeneration system), or both, the enzymes involved are expressed in cells of the same host as described above, the oxidative and/or the reductive reaction system would be separated from each other by the host cell membrane and, as a result, the above-described failure due to conjugation of the oxidized form coenzyme regeneration system and the reduced form coenzyme regeneration system does not occur, or becomes unlikely to occur; as a result, even if the coenzyme specificity of the enzyme involved in the coenzyme regeneration system is low, the reaction may proceed well.

On the other hand, it is preferable for streamlining the production process that the reaction be performed using a culture product of a recombinant microorganism prepared by introducing an oxidizing enzyme gene and a reducing enzyme gene into cells of the same host microorganism; a recombinant microorganism prepared by introducing, in addition to an oxidizing enzyme gene and a reducing enzyme gene, a gene for an enzyme having the capability of regenerating the reduced form coenzyme on which the reducing enzyme depends into the same host microorganism, or a recombinant microorganism prepared by introducing, in addition to an oxidizing enzyme gene and a reducing enzyme gene, a gene for an enzyme having the capability of regenerating the oxidized form coenzyme on which the oxidizing enzyme depends into the same host microorganism, or a treatment product of the culture product and the like. It is more preferable that the reaction be performed using a culture product of a recombinant microorganism prepared by introducing all enzyme genes for an oxidizing enzyme, an enzyme having the capability of regenerating the oxidized form coenzyme on which the oxidizing enzyme depends, a reducing enzyme, and an enzyme having the capability of regenerating the reduced form coenzyme on which the reducing enzyme depends into the same host microorganism, or a treatment product of the culture product and the like. This patent is also intended to provide such a recombinant microorganism.

Such a recombinant microorganism can be produced by incorporating 2 to 4 DNAs selected from the group

consisting of a DNA that encodes the oxidizing enzyme used, a DNA that encodes the reducing enzyme used, a gene that encodes an enzyme having the capability of regenerating the oxidized form coenzyme, and a DNA that encodes an enzyme having the capability of regenerating a reduced form coenzyme into the same vector, and introducing the vector into a host. Alternatively, such a recombinant microorganism can also be produced by incorporating these 2 to 4 kinds of DNA into a plurality of vectors in different incompatibility groups, respectively, and introducing these vectors into the same host.

The oxidizing enzyme, reducing enzyme, oxidized form coenzyme regeneration enzyme and reduced form coenzyme regeneration system enzyme used in the present invention may be totally or partially purified enzymes, and a culture product of a microorganism having the capability of producing these enzymes or a treatment product thereof can also be used. Here, "a culture product of a microorganism" means a culture broth containing cells or cultured cells; "a treatment product thereof" means, for example, a crude extract, a freeze-dried microbial cell, an acetone-dried microbial cell, or a milling product of the cell and the like. Furthermore, they can be used after being immobilized as the enzyme or cells as is by a commonly known means. The immobilization can be performed by a method obvious to those skilled in the art (for example, crosslinkage method, physical adsorption method, inclusion method and the like).

The above-described microorganism having the capability of producing an enzyme may be a wild strain or a mutant strain, or a recombinant prepared by inserting a DNA of the enzyme to a vector, and introducing the vector into a host.

Next, the secondary alcohol used or produced in the present invention is described. However, the gist of the present invention resides in, as described above, a method of producing an optically active secondary alcohol, comprising using in combination an oxidizing enzyme source and a reducing enzyme source having different coenzyme specificities and different enantio-selectivities, and further efficiently combining a coenzyme regeneration system with the oxidative reaction and the reductive reaction.

Therefore, the enantiomer mixture of secondary alcohol used in the present invention is not subject to limitation, as long as it is a compound having a secondary hydroxyl group as described above; as representative examples thereof, 1,2-diol and 2-alkanol can be mentioned.

As the 1,2-diol, a 1,2-diol represented by the general formula (1):

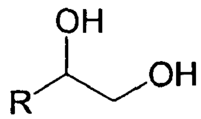

(1)

(wherein R represents an alkyl group having 1 to 10 carbon atoms, and optionally having a substituent, or an aryl group having 5 to 15 carbon atoms, and optionally having a substituent) can be mentioned.

As the 2-alkanol, a 2-alkanol represented by the general formula (2):

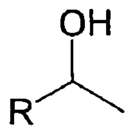

(2)

(wherein R represents an alkyl group having 1 to 10 carbon atoms, and optionally having a substituent, or an aryl group having 5 to 15 carbon atoms, and optionally having a substituent) can be mentioned.

The optically active secondary alcohol produced in the present invention is not subject to limitation, as long as it is a compound having a secondary hydroxyl group; an optically active 1,2-diol represented by the general formula (3):

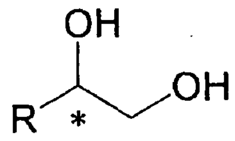

(3)

(wherein R is the same as above. * represents an asymmetric carbon) and an optically active 2-alkanol represented by the general formula (4):

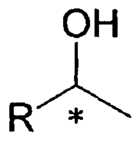

(4)

(wherein R is the same as above. * represents an asymmetric carbon) can be mentioned. In the foregoing general formulas (3) and (4), * represents an asymmetric carbon, whose absolute configuration may be the (R) form or the (S) form.

That is, an optically active 1,2-diol represented by the general formula (5):

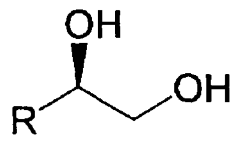

(5)

(wherein R is the same as above.), or the general formula (6):

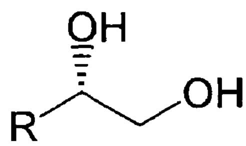

(6)

(wherein R is the same as above.), and an optically active 2-alkanol represented by the general formula (7):

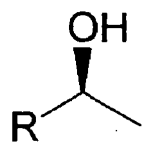

(7)

(wherein R is the same as above.), or the general formula (8):

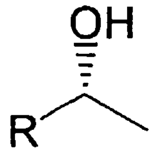

(8)

(wherein R is the same as above.), and derivatives thereof can be mentioned.

In the foregoing formulas (1) to (8), as the alkyl group having 1 to 10 carbon atoms, and optionally having a substituent, a methyl group, an ethyl group, an n-propyl group, an isopropyl group, an n-butyl group, a sec-butyl group, a tert-butyl group, an n-pentyl group, an n-hexyl group, an n-octyl, or a halomethyl group and the like can be mentioned. As the halomethyl group, a chloromethyl group, a bromomethyl group, or an iodomethyl group can be mentioned. As the aryl group having 5 to 15 carbon atoms, and optionally having a substituent, a phenyl group, an o-chlorophenyl group, an m-bromophenyl group, a p-fluorophenyl group, a p-nitrophenyl group, a p-cyanophenyl group, or a p-methoxyphenyl group and the like can be mentioned.

Specifically, as the 1,2-diol represented by the foregoing formula (1), (3), (5), or (6), 3-chloro-1,2-butanediol, 1,2-butanediol, 1,2-pentanediol, 1,2-hexanediol, 4-methyl-1,2-pentanediol, 1-phenyl-1,2-ethanediol and derivatives thereof can be mentioned.

Specifically, as the 2-alkanol represented by the foregoing formula (2), (4), (7), or (8), 2-butanol, 2-pentanol, 2-hexanol, 1-phenylethanol, 3-hydroxy-2-butanol and derivatives thereof can be mentioned.

Next, the oxidizing enzyme source and reducing enzyme source used in the present invention are described. In the present invention, as the oxidizing enzyme source and the reducing enzyme source, dehydrogenases classified under EC.1.1.1 are used. A combination of an oxidizing enzyme source and a reducing enzyme source may be selected as appropriate according to the desired secondary alcohol.

Enzymatic asymmetric reduction is one of the most useful techniques in the synthesis of an optically active alcohol, and dehydrogenases derived from various microorganisms or animal tissues have been reported along with their substrate specificities and enantio-selectivities. With reference to these pieces of information, an oxidizing enzyme source and a reducing enzyme source having an enantio-selectivity and a coenzyme specificity suitable for the method of the invention of this application may be selected.

If no known information is available on the desired secondary alcohol, an oxidizing enzyme source and a reduced form enzyme source can be selected as described below.

1) A ketone compound corresponding to the desired secondary alcohol, for example, a ketone compound represented by the general formula (9):

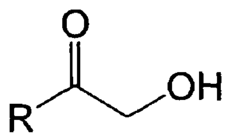

(9)

(wherein R is the same as above), or the general formula (10):

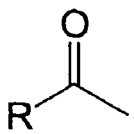

(10)

(wherein R is the same as above) is obtained or prepared.

2) Next, using various commercially available dehydrogenases or microorganisms, the ketone compound is reduced into a secondary alcohol, and the enantio-selectivity is confirmed.

3) Several kinds of enzyme sources exhibiting good (R) selectivity and several kinds of enzyme sources exhibiting

good (S) selectivity are selected, and their coenzyme specificities are confirmed.

4) On the basis of the enantio-selectivities and coenzyme specificities, and a combination of an oxidizing enzyme and a reducing enzyme is determined.

Oxidizing enzyme sources and reducing enzyme sources that can be used in the present invention are hereinafter described. As described above, the gist of the present invention resides in a method of producing an optically active secondary alcohol, comprising using in combination an oxidizing enzyme source and a reducing enzyme source having different coenzyme specificities and different enantio-selectivities, and further efficiently combining the oxidative reaction and the reductive reaction with a coenzyme regeneration system. Therefore, the present invention is not limited by any means by the oxidizing enzyme source and reducing enzyme source used.

Regarding oxidizing enzyme sources and reducing enzyme sources that can be used in the present invention, as an example of the enzyme source having the capability of selectively oxidizing an enantiomer of a 1,2-diol represented by the foregoing formula (5), and having specificity for NAD+, an enzyme source derived from a microorganism belonging to the genus *Cellulomonas* can be mentioned; preferably, an enzyme source derived from *Cellulomonas* sp. KNK0102 (WO05/123921) can be mentioned.

As the enzyme source having the capability of selectively oxidizing an enantiomer of a 1,2-diol represented by the foregoing formula (6), and having specificity for NAD+, an enzyme source derived from a microorganism selected from the group consisting of the genus *Candida* and the genus *Ochrobactrum* can be mentioned; preferably, an enzyme source derived from *Candida malis* NBRC10003 (WO01/005996) and an enzyme source derived from *Ochrobactrum sp.* KNKc71-3 can be mentioned.

As the enzyme source having the capability of selectively oxidizing an enantiomer of a 2-alkanol represented by the foregoing formula (7), and having specificity for NAD+, an enzyme source derived from a microorganism belonging to the genus *Ochrobactrum* can be mentioned; preferably, an enzyme source derived from *Ochrobactrum sp.* KNKc71-3 can be mentioned.

As the enzyme source having the capability of selectively oxidizing an enantiomer of a 2-alkanol represented by the foregoing formula (8), and having specificity for NAD+, an enzyme source derived from a microorganism belonging to the genus *Candida* can be mentioned; preferably, an enzyme source derived from *Candida malis* NBRC10003 can be mentioned.

As the enzyme source having the capability of reducing a ketone compound represented by the foregoing formula (9) into an enantiomer of a 1,2-diol represented by the foregoing formula (6), and having specificity for NAHPH, an enzyme source derived from a microorganism belonging to the genus *Candida* can be mentioned; preferably, an enzyme source derived from *Candida magnoriae* NBRC0705 (WO98/035025) can be mentioned.

As the enzyme source having the capability of reducing a ketone compound represented by the foregoing formula (9) into an enantiomer of a 1,2-diol represented by the foregoing formula (5), and having specificity for NADPH, an enzyme source derived from a microorganism belonging to the genus *Rhodotorula* can be mentioned; preferably, an enzyme source derived from *Rhodotorula glutinis* NBRC415 (WO03/093477) can be mentioned.

As the enzyme source having the capability of reducing a ketone compound represented by the foregoing formula (10) into an enantiomer of a 2-alkanol represented by the foregoing formula (8), and having specificity for NADPH, an enzyme source derived from a microorganism belonging to the genus *Candida* can be mentioned; preferably, an enzyme source derived from *Candida magnoriae* NBRC0705 can be mentioned.

As the enzyme source having the capability of reducing a ketone compound represented by the foregoing formula (10) into an enantiomer of a 2-alkanol represented by the foregoing formula (7), and having specificity for NADPH, a enzyme source derived from a microorganism belonging to the genus *Rhodotorula* can be mentioned; preferably, an enzyme source derived from *Rhodotorula glutinis* NBRC415 can be mentioned.

Combinations of an oxidizing enzyme source and a reducing enzyme source, suitable for the present invention, are described.

An oxidizing enzyme source capable of enantio-selectively oxidizing a 1,2-diol represented by the foregoing formula (5) to produce a ketone compound represented by the foregoing formula (9) is combined with a reducing enzyme source that enantio-selectively reduces the ketone compound (9) to produce a 1,2-diol represented by the foregoing formula (6). Likewise, an oxidizing enzyme source capable of enantio-selectively oxidizing the 1,2-diol (6) is combined with a reducing enzyme source capable of enantio-selectively reducing the ketone (9) to produce the 1,2-diol (5).

An oxidizing enzyme source capable of enantio-selectively oxidizing a 2-alkanol represented by the foregoing formula (7) to produce a ketone compound represented by the foregoing formula (10) is combined with a reducing enzyme source capable of enantio-selectively reducing the ketone compound (10) to produce the 2-alkanol (8). Likewise, an oxidizing enzyme source capable of enantio-selectively oxidizing the 2-alkanol (8) is combined with a reducing enzyme source capable of enantio-selectively reducing the ketone (10) to produce the 2-alkanol (7).

In all combinations described above, it is preferable that the oxidizing enzyme source and the reducing enzyme source have different coenzyme specificities. That is, if the oxidizing enzyme source is specific for NAD+, the reducing

enzyme source is specific for NADPH; if the oxidizing enzyme source is specific for NADP+, the reducing enzyme source is NADH-specific.

Particularly, a combination of an NAD+-specific oxidizing enzyme source and an NADPH-specific reducing enzyme source is preferable because it allows the use of an NADH oxidase in the oxidized form coenzyme regeneration system, and an NADP+-specific glucose dehydrogenase in the reduced form coenzyme regeneration system.

Some example combinations of an oxidizing enzyme source and a reducing enzyme source are given below, which, however, are not to be construed as limiting the present invention.

To produce an optically active 1,2-diol represented by the foregoing formula (6), for example, a combination of an oxidizing enzyme source derived from a microorganism belonging to the genus *Cellulomonas,* preferably *Cellulomonas* sp. KNK0102, and a reducing enzyme source derived from a microorganism belonging to the genus *Candida,* preferably *Candida magnoriae* NBRC0705, can be mentioned.

To produce an optically active 1,2-diol represented by the foregoing formula (5), for example, a combination of an oxidizing enzyme source derived from a microorganism belonging to the genus *Candida* or the genus *Ochrobactrum,* preferably *Candida malis* NBRC10003 or *Ochrobactrum sp.* KNKc71-3, and a reducing enzyme source derived from a microorganism belonging to the genus *Rhodotorula*, preferably *Rhodotorula glutinis* NBRC415, can be mentioned.

To produce an optically active 2-alkanol represented by the foregoing formula (8), for example, a combination of an oxidizing enzyme source derived from a microorganism belonging to the genus *Ochrobactrum,* preferably *Ochrobactrum sp.* KNKc71-3, and a reducing enzyme source derived from a microorganism belonging to the genus *Candida,* preferably *Candida magnoriae* NBRC0705, can be mentioned.

To produce an optically active 2-alkanol represented by the foregoing formula (7), for example, a combination of an oxidizing enzyme source derived from a microorganism belonging to the genus *Candida*, preferably *Candida malis* NBRC10003, and a reducing enzyme source derived from a microorganism belonging to the genus *Rhodotorula*, preferably *Rhodotorula glutinis* NBRC415, can be mentioned.

Of the above-described microorganisms, *Candida malis* NBRC1003, *Candida magnoriae* NBRC0705, and *Rhodotorula glutinis* NBRC415 have been stored at the NITE Biological Resource Center, Department of Biotechnology, National Institute of Technology and Evaluation (NBRC: 2-5-8, Kazusa-Kamatari, Kisarazu-shi, Chiba 292-0818), and are available from the organization.

*Cellulomonas* sp. KNK0102 was isolated and identified from soil by the present inventors, and how it was acquired and the like are described in WO05/123921.

The microorganism having the capability of producing the oxidizing enzyme or reducing enzyme used in the present invention may be any of a wild strain or a mutant strain. Alternatively, a microorganism derivatized by a genetic technique such as cell fusion or gene manipulation can also be used. A gene-manipulated microorganism that produces the above-described enzymes can be obtained by, for example, a method comprising a step for isolating and/or purifying these enzymes and determining a partial or the entire amino acid sequence of each enzyme, a step for obtaining a DNA sequence that encodes the enzyme on the basis of this amino acid sequence, a step for introducing this DNA to another host microorganism to yield a recombinant microorganism, and a step for culturing this recombinant microorganism to yield this enzyme (WO98/35025). As the host, bacteria, yeast, filamentous fungi and the like can be mentioned, and *Escherichia coli* is particularly preferable.

As examples of recombinant microorganisms transformed with a plasmid having a DNA that encodes the above-described oxidizing enzyme or reducing enzyme, the following recombinant microorganisms can be mentioned.

a) *Escherichia coli* HB101 (pTSCS) FERM BP-10024 transformed with the glycerol dehydrogenase (hereinafter referred to as dehydrogenase: oxidizing enzyme) gene derived from *Cellulomonas* sp. KNK0102 (WO05/123921).
b) *Escherichia coli* HB101 (pNTFP) FERM BP-7116 transformed with the dehydrogenase (oxidizing enzyme) gene derived from *Candida malis* NBRC10003 (WO01/005996).
c) *Escherichia coli* HB101 (pTSOB) FERM BP-10461 transformed with the dehydrogenase (oxidizing enzyme) derived from *Ochrobactrum* sp. KNKc71-3.
d) Escherichia *coli* HB101 (pNTS1) FERM BP-5834 transformed with the dehydrogenase (reducing enzyme) gene derived from *Candida magnoliae* IFO0705 (WO98/035025)
e) *Escherichia coli* HB101 (pNTRG) FERM BP-7857 transformed with the dehydrogenase (reducing enzyme) gene derived from *Rhodotorula glutinis* NBRC415 (WO03/093477).

The above-described recombinant microorganisms have been deposited under the foregoing respective accession numbers with the International Patent Organism Depositary, National Institute of Advanced Industrial Science and Technology (IPOD: Central 6, 1-1, Higashi, Tsukuba, Ibaraki, 305-8566).

[Deposition dates]

*Escherichia coli* HB101 (pTSCS) FERM BP-10024: May 12, 2004
*Escherichia coli* HB101 (pNTFP) FERM BP-7116: April 11, 2000
*Escherichia coli* HB101 (pTSOB) FERM BP-10461: November 30, 2005
*Escherichia coli* HB101 (pNTS1) FERM BP-5834: February 24, 1997
*Escherichia coli* HB101 (pNTRG) FERM BP-7857: January 22, 2002

In the present invention, a recombinant microorganism that simultaneously expresses 2 to 4 enzymes out of an oxidizing enzyme, an enzyme having the capability of regenerating an oxidized form coenzyme, a reducing enzyme, and a reduced form coenzyme as described above can be suitably used. A recombinant microorganism that produces these enzymes can be prepared by an ordinary gene recombination technology utilizing an oxidizing enzyme gene, a reducing enzyme gene, or a recombinant plasmid incorporating them, contained in the recombinant microorganisms mentioned in a) to e) above.
Details of the oxidizing enzyme gene or reducing enzyme gene, and recombinant plasmid, contained in the recombinant microorganisms mentioned in the foregoing a) to e), are described in the reference documents mentioned in the respective sections.

*Ochrobactrum sp.* KNKc71-3 is a microorganism isolated and identified from soil by the present inventors. The dehydrogenase (oxidizing enzyme) derived from *Ochrobactrum sp.* KNKc71-3 has been introduced to the foregoing recombinant microorganism *Escherichia coli* HB101 (pTSOB) FERM BP-10461 as the recombinant vector pTSOB shown in Figure 2. Those skilled in the art can isolate the recombinant vector pTSOB from the recombinant microorganism by a conventional method, and utilize the same for introducing an additional gene and the like.

The culture medium for the microorganism used as the enzyme source is not subject to limitation, as long as it allows the microorganism to grow. For example, an ordinary liquid medium comprising saccharides such as glucose and sucrose, alcohols such as ethanol and glycerol, fatty acids such as oleic acid and stearic acid and esters thereof, and oils such as rapeseed oil and soybean oil as carbon sources; ammonium sulfate, sodium nitrate, peptone, casamino acid, corn steep liquor, bran, yeast extract and the like as nitrogen sources; magnesium sulfate, sodium chloride, calcium carbonate, potassium monohydrogen phosphate, potassium dihydrogen phosphate and the like as inorganic salts; and malt extract, meat extract and the like as other nutrient sources, can be used. Cultivation is performed under aerobic conditions; usually, cultivation time is about 1 to 5 days, the pH of the medium is 3 to 9, and cultivation temperature is 10 to 50°C.

The reaction conditions for the deracemization of the present invention vary depending on the enzymes used, microorganism or a treatment product thereof, substrate concentration and the like; usually, the substrate concentration is about 0.1 to 100% by weight, preferably 1 to 60% by weight, the ratio of coenzyme NAD(P)+ to the substrate is 0.0001 to 100 mol%, preferably 0.0001 to 0.1 mol%, and the ratio of coenzyme NAD(P)H to the substrate is 0.0001 to 100 mol%, preferably 0.0001 to 0.1 mol%. The reaction can be carried out at a reaction temperature of 10 to 60°C, preferably 20 to 50°C, at a reaction pH of 4 to 9, preferably 5 to 8, and for a reaction time of 1 to 120 hours, preferably 1 to 72 hours.

The substrate may be added at one time or continuously. The reaction can be performed by a batch process or a continuous process. When the NAD+ regeneration capability of an oxidizing enzyme-producing microorganism or NADH oxidase is used for regenerating an oxidized form coenzyme, it is preferable that the reaction be performed in the presence of air or relatively pure oxygen under aerobic conditions. To facilitate the dissolution of oxygen in the reaction mixture, the reaction is preferably performed under shaking or stirring conditions. Furthermore, by performing the reaction under an increased pressure of more than atmospheric pressure, the solubility of oxygen in the reaction mixture may increase so that the reaction proceeds more efficiently.

The optically active secondary alcohol resulting from the deracemation reaction can be purified by a conventional method. For example, optically active 3-chloro-1,2-propanediol can be purified by making treatments such as centrifugation and filtration as required to remove the suspension of cells and the like when a microorganism and the like are used, and then extracting with an organic solvent such as ethyl acetate or toluene, removing the organic solvent under reduced pressure, and making a treatment such as distillation under reduced pressure or chromatography.

The present invention is hereinafter described in more detail by means of the following examples, which, however, are not to be construed as limiting the present invention.

## Examples

The present invention is hereinafter described in more detail by means of the following examples, which, however, are not to be construed as limiting the present invention.

(Example 1) Synthesis of (R)-3-chloro-1,2-propanediol (an oxidized form coenzyme regeneration system using *Escherichia coli* cells and a reduced form coenzyme regeneration system based on a glucose dehydrogenase were used in combination)

*E. coli* HB101 (pTSCS) FERM BP-10024 was inoculated to 50 ml of 2xYT medium (Bacto Trypton 1.6%, Bacto Yeast Extract 1.0%, NaCl 0.5%, pH 7.0) sterilized in a 500 ml capacity Sakaguchi flask, and subjected to shaking culture at 37°C for 18 hours. 50 ml of the above-described culture broth was centrifuged, and cells were harvested and suspended in 50 ml of 100 mM phosphate buffer solution (pH 8.0).

*E. coli* HB101 (pNTS1) FERM BP-5834 was inoculated to 50 ml of 2×YT medium (Trypepton 1.6%, yeast extract 1.0%, NaCl 0.5%, pH 7.0) sterilized in a 500 ml capacity Sakaguchi flask, and subjected to shaking culture at 37°C for 18 hours. 50 ml of this culture broth was centrifuged, and cells were harvested and suspended in 50 ml of 100 mM phosphate buffer solution (pH 8.0), and homogenized by sonication using a UH-50 model ultrasonic homogenizer (manufactured by SMT Company) to yield a cell-free extract.

1 ml of the above-described suspension of *E. coli* HB101 (pTSCS), 20 mg of racemic 3-chloro-1,2-propanediol, 1 ml of the above-described cell-free extract of E. *coli* HB101 (pNTS1), 10 U of glucose dehydrogenase (manufactured by Amano Enzyme Inc.), 0.2 mg of NADP+, and 40 mg of glucose were added to a stoppered test tube, and while adjusting to pH 8.0 with 2 M sodium hydroxide aqueous solution, this mixture was stirred at 30°C for 20 hours. After completion of the reaction, the reaction mixture was saturated with ammonium sulfate and then subjected to extraction with the addition of ethyl acetate, the 3-chloro-1,2-propanediol content remaining in the extract was analyzed by capillary gas chromatography, and the percent recovery (%) was calculated. After trifluoroacetylation, the above-described product was analyzed by capillary gas chromatography, and the optical purity (%e.e.) was calculated. As a result, the percent recovery was 90%, and the optical purity was 96.1% e.e.(R).

[Content analytical conditions]

Column: HP-5 30 m × 0.32 mm I.D. (manufactured by Agilent Technologies Company)
Detection: FID
Initial column temperature: 50°C
Final column temperature: 200°C
Heating speed: 6°C/minute
Injection temperature: 150°C
Detection temperature: 300°C
Carrier gas: helium (70 kPa)
Split ratio: 100/1
Detection time: 3-chloro-1,2-propanediol 10.2 minutes

[Optical purity analytical conditions]

Column: Chiradex G-PN (30 m × 0.25 mm) (manufactured by ASTEC Company)
Column temperature: 90°C
Injection temperature: 150°C
Detection temperature: 150°C
Carrier gas: helium (130 kPa)
Split ratio: 100/1
Detection time: R form 10.0 minutes, S form 10.6 minutes.

$$\text{Optical purity } (\%\text{e.e.}) = (A-B)/(A+B) \times 100$$

(A and B indicate the amounts of corresponding enantiomers and meet the requirement of A>B)

(Example 2) Synthesis of (R)-3-chloro-1,2-propanediol (an oxidized form coenzyme regeneration system using a hydrogen peroxide-producing NADH oxidase and a reduced form coenzyme regeneration system based on a glucose dehydrogenase were used in combination)

50 ml of the culture broth of E. *coli* HB101 (pTSCS) obtained in Example 1 was centrifuged, and cells were harvested and suspended in 50 ml of 100 mM phosphate buffer solution (pH 8.0). Thereafter, the cells were homogenized

by sonication using a UH-50 model ultrasonic homogenizer (manufactured by SMT Company) to yield a cell-free extract.

1 ml of the above-described cell-free extract of E. *coli* HB101 (pTSCS), 20 mg of racemic 3-chloro-1,2-propanediol, 0.2 mg of NAD+, 1 U of NADH oxidase (manufactured by SIGMA Company), 20 U of catalase (manufactured by SIGMA Company), and 1 ml of the cell-free extract of E. *coli* HB101 (pNTS1) obtained in Example 1, 10 U of glucose dehydrogenase (manufactured by Amano Enzyme Inc.), 0.2 mg of NADP+, and 40 mg of glucose were added to a stoppered test tube, and while adjusting to pH 8.0 with 2 M sodium hydroxide aqueous solution, this mixture was stirred at 30°C for 20 hours. After completion of the reaction, an analysis was performed by the method described in Example 1; as a result, the percent recovery was 67%, and the optical purity was 71.2% e.e.(R).

(Example 3) Synthesis of (R)-3-chloro-1,2-propanediol (an oxidized form coenzyme regeneration system using *Escherichia coli* cells and a reduced form coenzyme regeneration system based on a glucose dehydrogenase were used in combination)

500 ml of the culture broth of E. *coli* HB101 (pNTS1) obtained in Example 1 was centrifuged, and cells were harvested and suspended in 25 ml of 100 mM phosphate buffer solution (pH 7.0). Thereafter, the cells were homogenized by sonication using a UH-50 model ultrasonic homogenizer (manufactured by SMT Company) to yield a cell-free extract.

2 ml of the culture broth of E. *coli* HB101 (pTSCS) obtained in Example 1, 100 mg of racemic 3-chloro-1,2-propanediol, 200 μl of the above-described cell-free extract of *E. coli* HB101 (pNTS1), 10 U of glucose dehydrogenase (manufactured by Amano Enzyme Inc.), 0.2 mg of NADP+, and 80 mg of glucose were added to a stoppered test tube, and while adjusting to pH 7.0 with 2 M sodium hydroxide aqueous solution, this mixture was stirred at 30°C for 28 hours. After completion of the reaction, an analysis was performed by the method described in Example 1; as a result, the percent recovery was 93.2%, and the optical purity was 98.6% e.e.(R).

(Example 4) Synthesis of (S)-1,2-butanediol (an oxidized form coenzyme regeneration system using *Escherichia coli* cells and a reduced form coenzyme regeneration system based on a glucose dehydrogenase were used in combination)

Using racemic 1,2-butanediol, a deracemation reaction was performed by the same method as Example 3. After completion of the reaction, the reaction mixture was saturated with ammonium sulfate and then subjected to extraction with the addition of ethyl acetate, the 1,2-butanediol content remaining in the extract was analyzed by the method described in Example 1, and the percent recovery (%) was calculated. After trifluoroacetylation, the above-described product was analyzed by capillary gas chromatography, and the optical purity (% e.e.) was calculated. As a result, the percent recovery was 83%, and the optical purity was 99.2% e.e.(S).

[Optical purity analytical conditions]

Column: Chiradex G-TA (20 m × 0.25 mm) (manufactured by ASTEC Company)
Column temperature: 60°C
Injection temperature: 150°C
Detection temperature: 150°C
Carrier gas: helium (130 kPa)
Split ratio: 100/1
Detection time: 1,2-butanediol R form 5.0 minutes, S form 5.8 minutes.

(Example 5) Synthesis of (S)-1,3-butanediol (an oxidized form coenzyme regeneration system using *Escherichia coli* cells and a reduced form coenzyme regeneration system based on a glucose dehydrogenase were used in combination)

Using racemic 1,3-butanediol, a deracemation reaction was performed by the same method as Example 3. After completion of the reaction, an analysis was performed by the method described in Example 4; as a result, the percent recovery was 77.2%, and the optical purity was 72.3% e.e.(S).

(Example 6) Synthesis of (R)-3-chloro-1,2-propanediol (an oxidized form coenzyme regeneration system using *Escherichia coli* cells and a reduced form coenzyme regeneration system based on a glucose dehydrogenase were used in combination)

100 ml of the culture broth of E. *coli* HB101 (pTSCS) obtained in Example 1, 7 g of racemic 3-chloro-1,2-propanediol, 10 ml of the cell-free extract of *E. coli* HB101 (pNTS1) obtained in Example 3, 500 U of glucose dehydrogenase (manufactured by Amano Enzyme Inc.), 10 mg of NADP+, and 11.4 g of glucose were added to a 500 ml micro-jar, and while adjusting to pH 7.0 with 30% sodium hydroxide aqueous solution, this mixture was stirred at 30°C for 64

hours (350 rpm, aeration: 1 ml/min). After completion of the reaction, the reaction mixture was saturated with ammonium sulfate and subjected to extraction with the addition of ethyl acetate, and the extract was concentrated under reduced pressure, after which the concentrate was distilled under reduced pressure to yield 6.8 g of a colorless transparent oily substance. An analysis was performed by the method described in Example 1; as a result, the percent recovery was 96%, and the optical purity was 98.6% e.e.(R).

(Example 7) Synthesis of (S)-3-chloro-1,2-propanediol (an oxidized form coenzyme regeneration system using *Escherichia coli* cells and a reduced form coenzyme regeneration system based on a glucose dehydrogenase were used in combination)

*E. coli* HB101 (pNTFP) FERM BP-7116 was inoculated to 50 ml of 2×YT medium (Bacto Trypton 1.6%, Bacto Yeast Extract 1.0%, NaCl 0.5%, pH 7.0) sterilized in a 500 ml capacity Sakaguchi flask, and subjected to shaking culture at 37°C for 18 hours.

*E. coli* HB101 (pNTRG) FERM BP-7857 was inoculated to 50 ml of 2×YT medium (Bacto Trypton 1.6%, Bacto Yeast Extract 1.0%, NaCl 0.5%, pH 7.0) sterilized in a 500 ml capacity Sakaguchi flask, and subjected to shaking culture at 37°C for 18 hours. 50 ml of this culture broth was centrifuged, and cells were harvested and suspended in 2.5 ml of 100 mM phosphate buffer solution (pH 7.0). Thereafter, the cells were homogenized by sonication using a UH-50 model ultrasonic homogenizer (manufactured by SMT Company) to yield a cell-free extract.

2 ml of the above-described culture broth of *E. coli* HB101 (pNTFP), 60 mg of racemic 3-chloro-1,2-propanediol, 200 μl of the above-described cell-free extract of E. *coli* HB101 (pNTRG), 10 U of glucose dehydrogenase (manufactured by Amano Enzyme Inc.), 0.2 mg of NADP+, and 80 mg of glucose were added to a stoppered test tube, and while adjusting to pH 7.0 with 2 M sodium hydroxide aqueous solution, this mixture was stirred at 30°C for 20 hours. After completion of the reaction, an analysis was performed by the method described in Example 1; as a result, the percent recovery was 98.7%, and the optical purity was 43.8% e.e.(S).

(Example 8) Preparation of a recombinant vector comprising the NADH oxidase gene and preparation of a recombinant *Escherichia coli*

To express the water-producing NADH oxidase derived from *Streptococcus mutans* in *Escherichia coli,* a recombinant vector for transformation was prepared. First, a double-stranded DNA having an NdeI site added to the initiation site of the structural gene of the water-producing NADH oxidase, and also having a new stop codon and a PstI site added just after the original stop codon, was acquired by the method described below. Using a combination of synthetic primers, primer-1 (gaggatttgcatatgagtaaaatcgttattg: SEQ ID NO:1) and primer-2 (atgaaaacatgtgaattcccattgacatatc: SEQ ID NO:2), and a combination of synthetic primers, primer-3 (gatatgtcaatgggaattcacatgttttcat: SEQ ID NO: 3) and primer-4 (tttctgcagttatcatttagcttttaatgct: SEQ ID NO:4), with the plasmid pSSW61 comprising the water-producing NADH oxidase gene (see Biosci. Biotech. Biochem., 60(1), 39-43, 1996) as the template, PCR was performed to synthesize double-stranded DNAs (a) and (b), respectively. Furthermore, using the foregoing synthetic primers, primer-1 and primer-4, with a mixture of the double-stranded DNAs (a) and (b) obtained above as the template, PCR was performed to yield a double-stranded DNA. The DNA fragment obtained was digested with NdeI and PstI, and inserted to the NdeI and PstI sites downstream of the lac promoter in the plasmid pUCNT (see WO94/03613) to yield the recombinant plasmid pNTNX. The method of preparation and structure of pNTNX are shown in Figure 1. Using this recombinant vector pNTNX, *E. coli* HB101 (manufactured by Takara Shuzo Co., Ltd.) was transformed to yield E. *coli* HB101 (pNTNX).

(Example 9) Expression of a water-producing NADH oxidase by a recombinant *Escherichia coli*

The *E. coli* HB101 (pNTNX) obtained in Example 8 was cultured in a 2xYT medium (Bacto Trypton 1.6%(w/v), Bacto Yeast Extract 1.0%(w/v), NaCl 0.5%(w/v), pH 7.0) containing 100 μg/ml ampicillin and 0.8%(w/v) glycerin, and cells were harvested, after which they were suspended in 100 mM potassium phosphate buffer solution (pH 7.0) and homogenized by sonication to yield a cell-free extract. The NADH oxidase activity of this cell-free extract was measured as described below. The measurement of the NADH oxidase activity was performed by measuring the reduction in the absorbance at a wavelength of 340 nm at 30°C in 1.0 ml of a reaction mixture comprising 100 mM phosphate buffer solution (pH 7.0), 0.17 mM NADH, 0.2 mM EDTA, 0.02 mM FAD and 0.05 ml of an enzyme solution. Under these reaction conditions, the enzyme activity to oxidize 1 μmol of NADH into NAD+ in 1 minute was defined as 1 U. As a result, the specific activity of the NADH oxidase in the above-described cell-free extract was 30 U/mg protein.

(Example 10) Coenzyme selectivities of the glucose dehydrogenase derived from *Bacillus megaterium* and the glucose dehydrogenase derived from *Cryptococcus uniguttulatus*

Regarding the glucose dehydrogenase derived from *Bacillus megaterium* (manufactured by Amano Enzyme Inc.) and the NADP+-specific glucose dehydrogenase of *Cryptococcus uniguttulatus* (manufactured by SIGMA Company), activities on the coenzymes NAD+ and NADP+ were examined. To 980 μl of a reaction mixture comprising 100 mM phosphate buffer solution, 75 mM glucose, and 2 mM NAD+ or NADP+, the glucose dehydrogenase derived from *Bacillus megaterium* or the glucose dehydrogenase derived from *Cryptococcus uniguttulatus* was added; the reaction was performed at 30°C, and with the increase in the absorption at 340 nm as the index, the activity was measured. The results are shown in Table 1. As is evident from Table 1, the glucose dehydrogenase derived from *Bacillus megaterium* is non-specific for the coenzymes, whereas the glucose dehydrogenase derived from *Cryptococcus uniguttulatus* is specific for NADP+.
[Table 1]

| Enzyme | Relative activity | |
|---|---|---|
| | NADP+ | NAD+ |
| GDH derived from Bacillus megaterium | 100 | 98 |
| NADP-specific GDH derived from *Cryptococcus uniguttulatus* | 100 | 0 |

(In the Table, GDH represents glucose dehydrogenase.)

(Example 11) Synthesis of (R)-3-chloro-1,2-propanediol (an oxidized form coenzyme regeneration system using a water-producing NADH oxidase and a reduced form coenzyme regeneration system using an NADP+-specific/non-specific glucose dehydrogenase were used in combination)

Cells were harvested by centrifugation from the culture broth of E. *coli* HB101 (pTSCS) and culture broth of E. *coli* HB101 (pNTS1) obtained by the same method as Example 1, and the culture broth of E. *coli* HB101 (pNTNX) obtained in Example 9, and suspended in 100 mM phosphate buffer solution (pH 7.0). Thereafter, the cells were homogenized by sonication using a UH-50 model ultrasonic homogenizer (manufactured by SMT Company), the cell residue was removed by centrifugation, to prepare the NAD+-specific dehydrogenase (oxidizing enzyme) derived from *Cellulomonas* sp., the NADPH-specific dehydrogenase (reducing enzyme) derived from *Candida magnoliae* and the water-producing NADH oxidase (an enzyme having the capability of regenerating an oxidized form coenzyme) derived from *Streptococcus mutans,* respectively.
The following 3 kinds of deracemation reaction mixtures were prepared, 1 ml of each was shaken at 20°C in a test tube for 20 hours while its pH was adjusted to 7 with 5 M sodium hydroxide.

(1) 100 mM potassium phosphate buffer solution (pH 7.0), 30 mg of racemic 3-chloro-1,2-propanediol, 49 mg of glucose, 0.71 mg of NAD+, 0.15 mg of NADP+, 50 U of the foregoing NAD+-specific dehydrogenase (as 3-chloro-1,2-propanediol oxidation activity), 300 U of the foregoing NADPH-specific dehydrogenase (as 3-chloro-1-hydroxyacetone reduction activity), and 60 U of the foregoing water-producing NADH oxidase (glucose dehydrogenase not added).
(2) Composition (1) + the coenzyme non-specific glucose dehydrogenase derived from *Bacillus megaterium* (manufactured by Amano Enzyme Inc.) 35 U.
(3) Composition (1) + the NADP+-specific glucose dehydrogenase derived from *Cryptococcus uniguttulatus* (manufactured by SIGMA Company) 35 U.

[3-chloro-1,2-propanediol oxidizing activity measurement conditions]

Determined by measuring the increase in the absorbance at a wavelength of 340 nm at 30°C in 1.0 ml of a reaction mixture comprising 100 mM 3-chloro-1,2-propanediol, 0.17 mM NAD+, and 0.05 ml of an enzyme solution in 100 mM phosphate buffer solution (pH 7.0). Under these reaction conditions, the enzyme activity to oxidize 1 μmol of NADH into NAD+ in 1 minute was defined as 1 U.

[3-chloro-1-hydroxyacetone reducing activity measurement conditions]

Determined by measuring the decrease in the absorbance at a wavelength of 340 nm at 30°C in 1.0 ml of a reaction mixture comprising 20 mM 3-chloro-1-hydroxyacetone, 0.17 mM NADPH, and 0.05 ml of an enzyme solution in 100 mM phosphate buffer solution (pH 7.0). Under these reaction conditions, the enzyme activity to oxidize 1 μmol of NADH into NAD+ in 1 minute was defined as 1 U.

After completion of the reaction, an analysis was performed by the method described in Example 1, and the percent recovery and optical purity were calculated. The results are shown in Table 2.

[Table 2]

| | Percent recovery (%) | Optical purity (%) |
|---|---|---|
| No addition | 85 | 19.1 |
| GDH derived from *Bacillus megaterium* | 88 | 5.2 |
| NADP-specific GDH derived from *Cryptococcus uniguttulatus* | 94 | 100 |
| (In the Table, GDH represents glucose dehydrogenase.) | | |

(Example 12) Synthesis of (R)-3-chloro-1,2-propanediol (5% charge: an oxidized form coenzyme regeneration system using a water-producing NADH oxidase and a reduced form coenzyme regeneration system using an NADP+-specific glucose dehydrogenase were used in combination)

1 ml of a reaction mixture comprising 300 mM potassium phosphate buffer solution, 50 mg of racemic 3-chloro-1,2-propanediol, 81 mg of glucose, 0.71 mg of NAD+, 0.15 mg of NADP+, 50 U (as 3-chloro-1,2-propanediol oxidation activity) of the NAD+-specific dehydrogenase (oxidizing enzyme) derived from *Cellulomonas* sp. obtained in Example 11, 300 U (as 3-chloro-1-hydroxyacetone reduction activity) of the NADPH-specific dehydrogenase (reducing enzyme) derived from *Candida magnoriae,* 300 U of the water-producing NADH oxidase derived from *Streptococcus mutans* (an enzyme for regenerating an oxidized form coenzyme), and 100 U of the NADP+-specific glucose dehydrogenase derived from *Cryptococcus uniguttulatus* (manufactured by SIGMA Company: an enzyme for regenerating a reduced form coenzyme) was shaken in a test tube at 20°C and reacted for 22 hours, while adjusting to pH 7 with 5 M sodium hydroxide. After completion of the reaction, an analysis was performed by the method described in Example 1, and the percent recovery and the optical purity were calculated. As a result, (R)-3-chloro1,2-propanediol having an optical purity of 100% was produced at a percent recovery of 95%.

(Example 13) Synthesis of (S)-1,2-butanediol (an oxidized form coenzyme regeneration system using a water-producing NADH oxidase and a reduced form coenzyme regeneration system using an NADP+-specific glucose dehydrogenase were used in combination)

The reaction was performed for 23 hours by the same method as Example 12 except that racemic 1,2-butanediol was used in place of racemic 3-chloro1,2-propanediol. After completion of the reaction, an analysis was performed by the method described in Example 4, and the percent recovery and the optical purity were calculated. As a result, (S)-1,2-butanediol having an optical purity of 100% was produced at a percent recovery of 99.5%.

(Example 14) Synthesis of (S)-1-phenylethanol (an oxidized form coenzyme regeneration system using a water-producing NADH oxidase and a reduced form coenzyme regeneration system using an NADP+-specific glucose dehydrogenase were used in combination)

Cells were harvested by centrifugation from the culture broth of *E. coli* HB101 (pNTF) and culture broth of *E. coli* HB101 (pNTRG) obtained by the same method as Example 7, and suspended in 100 mM phosphate buffer solution (pH 7.0). Thereafter, the cells were homogenized by sonication using a UH-50 model ultrasonic homogenizer (manufactured by SMT Company), and the disruption residue was removed by centrifugation, to prepare the NAD+-specific dehydrogenase (oxidizing enzyme) derived from *Candida maris* and the NADPH-specific dehydrogenase (reducing enzyme) derived from *Rhodotorula glutinis,* respectively.

1 ml of a reaction mixture comprising 300 mM potassium phosphate buffer solution, 10 mg of racemic 1-phenylethanol, 7.4 mg of glucose, 0.71 mg of NAD+, 0.15 mg of NADP+, 40 U (as 1-phenylethanol oxidation activity) of the foregoing NAD+-specific dehydrogenase, 70 U (as acetophenone reduction activity) of the foregoing NADPH-specific dehydrogenase, 40 U of the water-producing NADH oxidase derived from *Streptococcus mutans* obtained in Example 8 (an enzyme for regenerating an oxidized form coenzyme), and 20 U of the NADP+-specific glucose dehydrogenase derived from *Cryptococcus uniguttulatus* (manufactured by SIGMA Company: an enzyme for regenerating a reduced form coenzyme) was shaken in a test tube at 20°C and reacted for 8 hours, while adjusting to pH 7 with 5 M sodium hydroxide. After 100 μl of the reaction mixture was saturated with ammonium sulfate, 1-phenylethanol was extracted with 700 μl of ethyl acetate, and the optical purity and percent recovery of 1-phenylethanol in this extract were analyzed by HPCL. As a result, (S) form-1-phenylethanol having an optical purity of 96.7% was produced at a percent recovery of 98%.

[1-phenylethanol oxidizing activity measurement conditions]

The same as the oxidizing activity measurement conditions described in Example 11 except that 1-phenylethanol was used in place of 3-chloro-1,2-propanediol.

[Acetophenone reducing activity measurement conditions]

The same as the reducing activity measurement conditions described in Example 11 except that acetophenone was used in place of 3-chloro-1-hydroxyacetone.

[1-phenylethanol HPLC analytical conditions]

Column: manufactured by Daicel Chemical Industries, Ltd., Chiralcell OD-H (0.46 × 25 cm)
Column temperature: 25°C
Eluent: n-hexane/2-propanol=9/1
Flow rate: 0.5 ml/minute
Elution time: (R) form-12.2 minutes, (S) form-13.3 minutes.

(Example 15) Preparation of a recombinant *Escherichia coli* transformed with the NADP+-specific glucol dehydrogenase of the *Cryptococcus uniguttulatus* JCM3687 strain

(1) Purification of enzyme

To a liquid medium consisting of the composition of 5 g of yeast extract (manufactured by Nihon Pharmaceutical Industrial Co., Ltd.), 7 g of polypeptone (manufactured by Nihon Pharmaceutical Industrial Co., Ltd.), 2.5 g of potassium dihydrogen phosphate, 1.0 g of ammonium chloride, 1.0 g of sodium chloride, 0.33 g of calcium chloride dihydrate, 0.0005 g of ferric sulfate heptahydrate, 0.5 g of magnesium sulfate heptahydrate, and 10 g of sucrose (all per liter), 2 ml of a culture broth of the *Cryptococcus uniguttulatus* JCM3687 strain, pre-cultured using the same medium in advance, was aseptically inoculated; the strain was subjected to shaking culture at 30°C for 50 hours. From 500 ml of the culture broth obtained, cells were harvested by centrifugation and washed with 50 mM phosphate buffer solution (pH 7.0), after which they were suspended in 200 ml of the same buffer solution. After this cell culture broth was homogenized using a Mini-Bead Beader (manufactured by BIOSPEC Company), the cell residue was removed by centrifugation to acquire 250 ml of a cell-free extract.

To this cell-free extract, while stirring with a stirrer under ice cooling, a predetermined amount of ammonium sulfate was added; the protein precipitated by ammonium sulfate (70-100% saturation) was collected by centrifugation. The protein obtained was dissolved in 50 mM phosphate buffer solution (pH 7.0) and dialyzed against the same buffer solution, after which this was applied to a column of CIM DEAE-8 (manufactured by BIA Separations Company), previously equilibrated with the same buffer solution, to adsorb the enzyme, and the active fraction was eluted by a linear density gradient of sodium chloride from 0 M to 1.0 M. The enzyme solution obtained was dialyzed against 50 mM phosphate buffer solution (pH 7.0) and applied to a column of 6 ml of ResourceQ (manufactured by Amersham Biosciences K.K.), previously equilibrated with the same buffer solution, to adsorb the enzyme, and the active fraction was eluted by a linear density gradient of sodium chloride from 0 M to 0.5 M. To the enzyme solution obtained, ammonium sulfate was added to obtain a concentration of 1.5 M; then, the solution was applied to a column of ResourcePHE (manufactured by Amersham Biosciences K.K.), previously equilibrated with a phosphate buffer solution comprising 1.5 M ammonium sulfate (pH 7.0), and the effluent fraction was collected as the active fraction.

When this active fraction was subjected to non-denatured polyacrylamide gel electrophoresis, a single band was formed. When this enzyme was subjected to SDS-polyacrylamide gel electrophoresis, a single band corresponding to a molecular weight of 58000 was formed.

(2) Cloning of glucose dehydrogenase

After denaturation in the presence of 8 M urea, the purified glucose dehydrogenase of the *Cryptococcus uniguttulatus* JCM3687 strain was digested with the lysyl endopeptidase derived from *Achromobacter* (manufactured by Wako Pure Chemical Industries, Ltd.), and the sequence of the peptide fraction obtained was determined by the Edman method. Considering the DNA sequence deduced from this amino acid sequence, 2 kinds of PCR primers, primer-5 (gagaagcagc acaagatyaargayca: SEQ ID NO:5) and primer-6 (catgtgrgcr agngargtraaytg: SEQ ID NO:6), were synthesized.

100 μl of a buffer solution for ExTaq comprising 50 pmol of each of the above-described two kinds of primers, 800 ng of chromosome DNA, 20 nmol of each dNTP, and 2.0 U of ExTaq (manufactured by Takara Bio Inc.) was prepared, thermal denaturation (94°C, 30 seconds), annealing (55°C, 30 seconds), and elongation reaction (72°C, 1 minute) were performed in 25 cycles, the about 750-base amplified fragment obtained was subcloned into pT7Blue-2 Vector (manufactured by Novagen Inc.), and its sequence was determined (SEQ ID NO:7)

Next, to determine the full length base sequence and the like of the cDNA that encodes the glucose dehydrogenase, on the basis of the base sequence determined here (SEQ ID NO:7), two kinds of gene sequence-specific primers, primer-7 (agttggccgagtacgttcagggagcgtatga: SEQ ID NO:8) and primer-8 (ggaaagcctc atcctcgtcatacgctccctg: SEQ ID NO:9) were synthesized.

To 60 ml of the medium described in the foregoing section (1), 3 ml of a culture broth of the *Cryptococcus uniguttulatus* JCM3687 strain, pre-cultured using the same medium in advance, was aseptically inoculated; the strain was subjected to shaking culture at 30°C for 6 hours. Obtained from the cultured cells using the RNAgents Total RNA Isolation System (manufactured by Promega K.K.) was 424 μg of total RNA. The total RNA obtained was purified using an Oligotex-dT30 column (manufactured by Takara Bio Inc.) to yield 4 μg of mRNA. Using 200 ng of the mRNA obtained, by means of the GeneRacer Kit (manufactured by Invitrogen Japan K.K), a cDNA was prepared by the method described in the protocol.

100 μl of a buffer solution for Pyrobest DNA Polymerase comprising 2.5 μl of the cDNA solution obtained, 50 pmol of each of the foregoing primer-7 and the GeneRacer3' nested primers attached to the GeneRacer Kit (primer-9, cgctacgtaacggcatgacagtg: SEQ ID NO:10) or 50 pmol of each of the foregoing primer-8 and the GeneRacer5' nested primer attached to the GeneRacer Kit (primer-10, ggacactgacatggactgaaggagta: SEQ ID NO:11), 20 nmol of each dNTP, and 2.5 U of Pyrobest DNA Polymerase (manufactured by Takara Bio Inc.), was prepared, thermal denaturation (96°C, 20 seconds), annealing and elongation reaction (72°C, 90 seconds) were performed in 4 cycles, subsequently thermal denaturation (96°C, 20 seconds), annealing and elongation reaction (70°C, 90 seconds) were performed in 4 cycles, still subsequently thermal denaturation (96°C, 20 seconds), annealing (58°C, 30 seconds), and elongation reaction (72°C, 90 seconds) were performed in 20 cycles, and the reaction product was cooled to 4°C, after which each amplified cDNA was identified by agarose gel electrophoresis. Each amplified cDNA was extracted using the QIAquick Gel Extraction Kit (manufactured by QIAGEN K.K.) and subcloned into the pCR4Blunt-TOPO vector (manufactured by Invitrogen Japan K.K), and sequencing of the amplified DNA was performed to determine the full length base sequence of the cDNA that encodes the glucose dehydrogenase. The full length base sequence and the deduced amino acid sequence encoded by the DNA are shown by SEQ ID NO:12 and 13, respectively, in the

sequence listing.

(3) Preparation of recombinant vector comprising the glucose dehydrogenase gene

To express the glucose dehydrogenase in *Escherichia coli,* a recombinant vector for use in transformation was prepared. First, the gene to be inserted in the vector was prepared as described below. On the basis of the base sequence determined in the section (2) above, primer-11 (acagacctgcccatatgtcgagca: SEQ ID NO:14), which has an NdeI site added to the initiation codon portion of the glucose dehydrogenase structural gene, and primer-12 (ggacggcgtctagatttactgcaaa: SEQ ID NO:15), which has an XbaI site added just after the stop codon, were synthesized.

100 μl of a buffer solution for Pyrobest DNA Polymerase comprising 50 pmol of each of the above-described 2 kinds of primers, 2.5 μl of the cDNA solution prepared in the section (2) above as the template, and 2.5 U of Pyrobest DNA Polymerase, was prepared, thermal denaturation (98°C, 10 seconds), annealing (57°C, 30 seconds), and elongation reaction (72°C, 3 minutes) were performed in 25 cycles, and the amplified DNA fragment obtained was digested with NdeI and XbaI and inserted to the NdeI and XbaI sites downstream of the lac promoter of the plasmid pUCNT (see WO94/03613). The plasmid obtained was designated as pNTGDH-J3687.

(4) Preparation of recombinant *Escherichia coli*

The *Escherichia coli* HB101 strain was transformed with the foregoing plasmid. After the transformant obtained

was cultured in a 2xYT medium (Bacto Trypton 1.6%(w/v), Bacto Yeast Extract 1.0%(w/v), NaCl 0.5%(w/v), pH 7.0) containing 5-0 μg/ml ampicillin, cells were collected by centrifugation and suspended in 50 mM phosphate buffer solution (pH 7.0), after which they were homogenized by sonication to yield a cell-free extract. This cell-free extract was treated with SDS and subjected to SDS-polyacrylamide gel electrophoresis; as a result, a band of the enzyme protein was identified at a position for a molecular weight of 58000.

The recombinant *Escherichia coli* obtained is called *Escherichia coli* HB101 (pNTGDH-J3687). *Escherichia coli* HB101 (pNTGDH-J3687) has been deposited under the accession number FERM P-20374 with the International Patent Organism Depositary, National Institute of Advanced Industrial Science and Technology (IPOD: Central 6, 1-1, Higashi, Tsukuba, Ibaraki, 305-8566) (Deposition date: January 21, 2005).

(Example 16) Synthesis of (R)-3-chloro-1,2-propanediol (an oxidized form coenzyme regeneration system using a water-producing NADH oxidase and a reduced form coenzyme regeneration system using the NADP+-specific glucose dehydrogenase derived from the JCM3687 strain were used in combination)

The *Escherichia coli* HB101 (pNTGDH-J3687) FERM P-20374 obtained in Example 15 was inoculated to 50 ml of a 2×YT medium (Bacto Trypton 1.6%(w/v), Bacto Yeast Extract 1.0%(w/v), NaCl 0.5%(w/v), pH 7.0) containing 50 μg/ml ampicillin and cultured in a 500 ml capacity Sakaguchi flask at 30°C for 30 hours. Cells were collected by centrifugation, suspended in 5 ml of 100 mM potassium phosphate buffer solution (pH 7.0), and homogenized by sonication to yield a cell-free extract, and this was used in the following reaction as the NADP+-specific glucose dehydrogenase source.

1 ml of a reaction mixture comprising 300 mM potassium phosphate buffer solution, 50 mg of racemic 3-chloro-1,2-propanediol, 81 mg of glucose, 0.71 mg of NAD+, 0.15 mg of NADP+, 50 U (as 3-chloro 1,2-propanediol oxidation activity) of the NAD+-specific dehydrogenase (oxidizing enzyme) derived from *Cellulomonas* sp. obtained in Example 11, 300 U (as 3-chloro-1-hydroxyacetone reduction activity) of the NADPH-specific dehydrogenase (reducing enzyme) derived from *Candida magnoriae,* 300 U of the water-producing NADH oxidase derived from *Streptococcus mutans* obtained by the same method as Example 9 (an enzyme for regenerating an oxidized form coenzyme), and 50 U of the above-described NADP+-specific glucose dehydrogenase derived from *Cryptococcus uniguttulatus* JCM3687 (an enzyme for regenerating a reduced form coenzyme), was shaken in a test tube at 20°C, and while adjusting to pH 7 with 5 M sodium hydroxide, they were reacted for 20 hours. After completion of the reaction, an analysis was performed by the method described in Example 1, and the percent recovery and the optical purity were calculated. As a result, (R)-3-chloro1,2-propanediol having an optical purity of 100% was produced at a percent recovery of 96%.

(Example 17) Synthesis of (R)-3-chloro-1,2-propanediol (an oxidized form coenzyme regeneration system using a water-producing NADH oxidase and a reduced form coenzyme regeneration system using the NADP+-specific glucose dehydrogenase derived from the JCM3687 strain were used in combination: reaction in a small incubator)

120 ml of a reaction mixture comprising 300 mM potassium phosphate buffer solution, 12 g of racemic 3-chloro-1,2-propanediol, 16 g of glucose, 12 mg of NAD+, 12 mg of NADP+, 3600 U (as 3-chlorol,2-propanediol oxidation activity) of the NAD+-specific dehydrogenase (oxidizing enzyme) derived from *Cellulomonas* sp. obtained in the same manner as Example 11, 12000 U (as 3-chloro-1-hydroxyacetone reduction activity) of the NADPH-specific dehydrogenase (reducing enzyme) derived from *Candida magnoriae,* 48000 U of the water-producing NADH oxidase derived from *Streptococcus mutans* obtained in the same manner as Example 9 (an enzyme for regenerating an oxidized form coenzyme), and 3600 U of the NADP+-specific glucose dehydrogenase derived from *Cryptococcus uniguttulatus* JCM3687 obtained in the same manner as Example 16 (manufactured by SIGMA Company: an enzyme for regenerating a reduced form coenzyme), was placed in a 250-ml capacity small incubator (Mitsuwa Rikagaku Kogyo Co., Ltd.), and while adjusting to pH 7.5 with 7.5 N sodium hydroxide aqueous solution, they were reacted at 20°C under conditions of aeration (1 vvm) and stirring (900 rpm) for 34 hours. As a result, (R)-3-chloro-1,2-propanediol having an optical purity of 100% was produced at a percent recovery of 96%.

(Example 18) Preparation of a recombinant vector comprising the NAD+-specific dehydrogenase gene derived from *Cellulomonas* sp. and the water-producing NADH oxidase gene derived from *Streptococcus mutans,* and a recombinant *Escherichia coli* (2-enzyme co-producing bacterium)

To co-produce 2 enzymes, the NAD+-specific dehydrogenase (oxidizing enzyme) derived from *Cellulomonas* sp. and the water-producing NADH oxidase derived from *Streptococcus mutans* (an enzyme for regenerating an oxidized form coenzyme) in *Escherichia coli,* a recombinant vector for use in transformation was prepared.

First, a double-stranded DNA having a BamHI site and the Shine-Dalgarno sequence (hereinafter abbreviated SD sequence) added to the initiation site of the structural gene of the water-producing NADH oxidase derived from

*Streptococcus mutans,* and also having a new stop codon and a PstI site added just after the original stop codon, was acquired by the method described below.

Using primer-13 (cgcggatcctaaggaggttaacaatgagtaaaatcgttattgttggagc: SEQ ID NO:16) and primer-14 (gcatgcctgcagttatcatttagcttt: SEQ ID NO:17), with the plasmid pNTNX comprising the water-producing NADH oxidase gene derived from *Streptococcus mutans,* obtained in Example 8, as the template, PCR was performed to yield a double-stranded DNA. This double-stranded DNA was digested with BamHI and PstI and inserted to the BamHI and PstI sites of the plasmid pTSCS comprising the dehydrogenase gene derived from *Cellulomonas* sp. (see WO05/123921), whereby the recombinant vector pTSCSNX was obtained. *E. coli* HB101 (manufactured by Takara Shuzo Co., Ltd.) was transformed with this recombinant vector pTSCSNX to yield *E. coli* HB101 (pTSCSNX), a bacterium that co-produces two enzymes, the dehydrogenase derived from *Cellulomonas* sp. and the water-producing NADH oxidase derived from *Streptococcus mutans.*

(Example 19) Preparation of a vector comprising the NADPH-specific dehydrogenase gene derived from *Candida magnoriae* and the NADP+-specific glucose dehydrogenase gene derived from *Cryptococcus uniguttulatus* JCM3687, and preparation of a recombinant expression bacterium (2-enzyme co-producing bacterium)

To co-produce 2 enzymes, the NADPH-specific dehydrogenase (reducing enzyme) derived from *Candida magnoriae* and the NADP+-specific glucose dehydrogenase derived from *Cryptococcus uniguttulatus* (an enzyme for regenerating a reduced form coenzyme) in *Escherichia coli,* a recombinant vector for use in transformation was prepared.

First, a double-stranded DNA having an XbaI site and the SD sequence added to the initiation site of the NADP+-specific glucose dehydrogenase gene derived from *Cryptococcus uniguttulatus,* and also having a HindIII site added after the stop codon, was acquired by the method described below. Using primer-15 (tgtctagacacacaggaaacacatatgtcgagcaccg: SEQ ID NO:18) and primer-16 (agtaagcttatttactgcaaaccagccgtgtatccaaac: SEQ ID NO:19), with the plasmid pNTGDH-J3687 comprising the NADP+-specific glucose dehydrogenase gene derived from *Cryptococcus uniguttulatus* (see Example 15) as the template, PCR was performed, to yield a double-stranded DNA. This double-stranded DNA was digested with XbaI and HindIII and inserted to the XbaI and HindIII sites of the plasmid pNTS1 comprising the dehydrogenase gene derived from *Candida magnoriae* (see WO98/035025), whereby the recombinant vector pNTS1G-J was obtained. *E. coli* HB101 (manufactured by Takara Shuzo Co., Ltd.) was transformed with this recombinant vector pNTS1G-J to yield *E. coli* HB101 (pNTS1G-J), a bacterium that co-produces 2 enzymes, the dehydrogenase derived from *Candida magnoriae* and the NADP+-specific glucose dehydrogenase derived from *Cryptococcus uniguttulatus:*

(Example 20) Synthesis of (R)-3-chloro-1,2-propanediol (2-enzyme co-producing bacteria were used in combination)

Each of the 2-enzyme co-producing bacteria *E. coli* HB101 (pTSCSNX) and *E. coli* HB101 (pNTS1G-J) obtained in Examples 18 and 19 was inoculated to 50 ml of 2xYT medium (Bacto Trypton 1.6%, Bacto Yeast Extract 1.0%, NaCl 0.5%, pH 7.0) sterilized in a 500 ml capacity Sakaguchi flask and subjected to shaking culture at 30°C for 36 hours. 50 ml of a culture broth of each of the above-described *E. coli* HB101 (pTSCSNX) and *E. coli* HB101 (pNTS1G-J) was centrifuged, and cells were harvested and suspended in 4 ml of 100 mM phosphate buffer solution (pH 7.0).

1 ml of a reaction mixture comprising 80 μl of the above-described cell suspension of each of *E. coli* HB101 (pTSCSNX) and *E. coli* HB101 (pNTS1G-J), 300 mM potassium phosphate buffer solution, 70 mg of racemic 3-chloro-1,2-propanediol, 114 mg of glucose, 1 mg of NAD+, and 1 mg of NADP+, was shaken in a test tube at 20°C, and while adjusting to pH 7 with 5 M sodium hydroxide, they were reacted for 20 hours. After completion of the reaction, an analysis was performed by the method described in Example 1, and the percent recovery and the optical purity were calculated. As a result, (R)-3-chloro-1,2-propanediol having an optical purity of 100% e.e. was produced at a percent recovery of 97%.

(Example 21) Preparation of a recombinant vector comprising the glycerol dehydrogenase gene derived from *Cellulomonas* sp., the dehydrogenase gene derived from *Candida magnoriae,* and the glucose dehydrogenase gene derived from *Cryptococcus uniguttulatus,* and a recombinant *Escherichia coli* (3-enzyme co-producing bacterium)

To co-produce 3 enzymes, the NAD+-specific dehydrogenase (oxidizing enzyme) derived from *Cellulomonas* sp., the NADPH-specific dehydrogenase (reducing enzyme) derived from *Candida magnoriae,* and the NADP+-specific glucose dehydrogenase derived from *Cryptococcus uniguttulatus* (an enzyme for regenerating a reduced form coenzyme) in *Escherichia coli,* a recombinant vector for use in transformation was prepared.

First, a double-stranded DNA having an EcoRI site and the SD sequence added to the initiation site of the structural gene of the dehydrogenase derived from *Cellulomonas* sp., and also having a new stop codon and an XbaI site added just after the original stop codon, was acquired by the method described below. Using primer-17 (aagccgaattctaaggaggttaacaatgtccgaggttcccgtccg: SEQ ID NO:20) and primer-18 (ttgcgtctagattatcagtgggcggtgtgcttga:

SEQ ID NO:21), with the plasmid pTSCS comprising the glycerol dehydrogenase gene derived from *Cellulomonas* sp. (see WO05/123921) as the template, PCR was performed to yield a double-stranded DNA. This double-stranded DNA was digested with EcoRI and XbaI and inserted to the EcoRI and XbaI sites of the plasmid pNTS1 comprising the dehydrogenase gene derived from *Candida magnoriae* (see WO98/035025), whereby the recombinant vector pNTSICS was obtained.

Next, a double-stranded DNA having an XbaI site and the SD sequence added to the initiation site of the structural gene of the NADP+-specific glucose dehydrogenase derived from *Cryptococcus uniguttulatus.,* and also having a HindIII site added just after the stop codon, was acquired by the method described below. Using primer-19 (aagcctctagataaggaggttaacaatgtcgagcaccgaatttca: SEQ ID NO:22) and primer-20 (ttgcgaagcttttagggaagcgtgtagccac: SEQ ID NO:23), with the plasmid pNTGDH-J3687 comprising the NADP+-specific glucose dehydrogenase gene derived from *Cryptococcus uniguttulatus* (see Example 15) as the template, PCR was performed to yield a double-stranded DNA. This double-stranded DNA was digested with XbaI and HindIII and inserted to the XbaI and HindIII sites of the above-described pNTS1CS, whereby the recombinant vector pNTS1CSGP was obtained.

Using this recombinant vector pNTS1CSGP, E. *coli* HB101 (manufactured by Takara Shuzo Co., Ltd.) was transformed, to yield E. *coli* HB101 (pNTS1CSGP), a 3-enzyme co-producing bacterium.

(Example 22) Synthesis of (R)-3-chloro-1,2-propanediol (a 3-enzyme co-producing bacterium and an oxidized form coenzyme regeneration system using a water-producing NADH oxidase were used in combination)

The 3-enzyme co-producing bacterium *E. coli* HB101 obtained in Example 21 (pNTS1CSGP) was inoculated to 50 ml of 2xYT medium (Bacto Trypton 1.6%, Bacto Yeast Extract 1.0%, NaCl 0.5%, pH 7.0) sterilized in a 500 ml capacity Sakaguchi flask, and subjected to shaking culture at 30°C for 36 hours. 50 ml of the above-described culture broth was centrifuged, and cells were harvested and suspended in 4 ml of 100 mM phosphate buffer solution (pH 7.0) (12.5 fold concentrated cells).

1 ml of a reaction mixture comprising 80 μl of the above-described concentrated cells, 300 mM potassium phosphate buffer solution, 70 mg of racemic 3-chloro-1,2-propanediol, 114 mg of glucose, 1 mg of NAD+, 1 mg of NADP+, and 300 U of the water-producing NADH oxidase derived from *Streptococcus mutans* obtained in Example 9, was shaken in a test tube at 20°C, and while adjusting to pH 7 with 5 M sodium hydroxide, they were reacted for 21 hours. After completion of the reaction, an analysis was performed by the method described in Example 1, and the percent recovery and the optical purity were calculated. As a result, (R)-3-chloro-1,2-propanediol having an optical purity of 100% e.e. was produced at a percent recovery of 100%.

(Example 23) Synthesis of (S)-1,2-propanediol (a 3-enzyme co-producing bacterium and an oxidized form coenzyme regeneration system using a water-producing NADH oxidase were used in combination)

1 ml of a reaction mixture comprising 80 μl of the concentrated cells obtained in Example 22, 300 mM potassium phosphate buffer solution, 10 mg of racemic 1,2-propanediol, 40 mg of glucose, 1 mg of NAD+, 1 mg of NADP+, and 300 U of the water-producing NADH oxidase derived from *Streptococcus mutans* obtained in Example 9, was shaken in a test tube at 20°C, and while adjusting to pH 7 with 5 M sodium hydroxide, they were reacted for 21 hours. After completion of the reaction, the reaction mixture was saturated with ammonium sulfate and then subjected to extraction with the addition of ethyl acetate, the 1,2-propanediol content remaining in the extract was analyzed by capillary gas chromatography, and the percent recovery was calculated. After trifluoroacetylation, the above-described product was analyzed by capillary gas chromatography, and the optical purity were calculated. As a result, (S)-1,2-propanediol having an optical purity of 100% e.e. was produced at a percent recovery of 98%.

[Content analytical conditions]

Column: HP-5 30 m × 0.32 mm I.D. (manufactured by Agilent Technologies Company)
Detection: FID
Column temperature: 70°C
Injection temperature: 150°C
Detection temperature: 150°C
Carrier gas: helium (150 kPa)
Split ratio: 100/1

[Optical purity analytical conditions]

Column: Chiradex G-PN (30 m × 0.25 mm) (manufactured by ASTEC Company)

Column temperature: 70°C
Injection temperature: 150°C
Detection temperature: 150°C
Carrier gas: helium (130 kPa)
Split ratio: 100/1

(Example 24) Synthesis of (S)-1,2-butanediol (a 3-enzyme co-producing bacterium and an oxidized form coenzyme regeneration system using a water-producing NADH oxidase were used in combination)

1 ml of a reaction mixture comprising 80 μl of the concentrated cells obtained in Example 22, 300 mM potassium phosphate buffer solution, 10 mg of racemic 1,2-butanediol, 40 mg of glucose, 1 mg of NAD+, 1 mg of NADP+, and 300 U of the water-producing NADH oxidase derived from *Streptococcus* mutans obtained in Example 9, was shaken in a test tube at 20°C, and while adjusting to pH 7 with 5 M sodium hydroxide, they were reacted for 5 hours. After completion of the reaction, an analysis was performed by the method described in Example 23, and the percent recovery and the optical purity were calculated. As a result, (S)-1,2-butanediol having an optical purity of 100% e.e. was produced at a percent recovery of 98%.

(Example 25) Synthesis of (S)-1,2-pentanediol (a 3-enzyme co-producing bacterium and an oxidized form coenzyme regeneration system using a water-producing NADH oxidase were used in combination)

1 ml of a reaction mixture comprising 80 μl of the concentrated cells obtained in Example 22, 300 mM potassium phosphate buffer solution, 10 mg of racemic 1,2-pentanediol, 40 mg of glucose, 1 mg of NAD+, 1 mg of NADP+, and 300 U of the water-producing NADH oxidase derived from *Streptococcus mutans* obtained in Example 9, was shaken in a test tube at 20°C, and while adjusting to pH 7 with 5 M sodium hydroxide, they were reacted for 2 hours. After completion of the reaction, an analysis was performed by the method described in Example 23, and the percent recovery and the optical purity were calculated. As a result, (S)-1,2-pentanediol having an optical purity of 100% e.e. was produced at a percent recovery of 96%.

(Example 26) Synthesis of (S)-1,2-hexanediol (a 3-enzyme co-producing bacterium and an oxidized form coenzyme regeneration system using a water-producing NADH oxidase were used in combination)

1 ml of a reaction mixture comprising 80 μl of the concentrated cells obtained in Example 22, 300 mM potassium phosphate buffer solution, 10 mg of racemic 1,2-hexanediol, 40 mg of glucose, 1 mg of NAD+, 1 mg of NADP+, and 300 U of the water-producing NADH oxidase derived from *Streptococcus mutans* obtained in Example 9, was shaken in a test tube at 20°C, and while adjusting to pH 7 with 5 M sodium hydroxide, they were reacted for 5 hours. After completion of the reaction, an analysis was performed by the method described in Example 23, and the percent recovery and the optical purity were calculated. As a result, (S)-1,2-hexanediol having an optical purity of 100% e.e. was produced at a percent recovery of 98%.

(Example 27) Synthesis of (S)-4-methyl-1,2-pentanediol (a 3-enzyme co-producing bacterium and an oxidized form coenzyme regeneration system using a water-producing NADH oxidase were used in combination)

1 ml of a reaction mixture comprising 80 μl of the concentrated cells obtained in Example 22, 300 mM potassium phosphate buffer solution, 10 mg of racemic 4-methyl-1,2-pentanediol, 40 mg of glucose, 1 mg of NAD+, 1 mg of NADP+, and 300 U of the water-producing NADH oxidase derived from *Streptococcus mutans* obtained in Example 9, was shaken in a test tube at 20°C, and while adjusting to pH 7 with 5 M sodium hydroxide, they were reacted for 27 hours. After completion of the reaction, an analysis was performed by the method described in Example 23, and the percent recovery and the optical purity were calculated. As a result, (S)-4-methyl-1,2-pentanediol having an optical purity of 81.3% e.e. was produced at a percent recovery of 100%.

(Example 28) Synthesis of (S)-1-phenyl-1,2-ethanediol (a 3-enzyme co-producing bacterium and an oxidized form coenzyme regeneration system using a water-producing NADH oxidase were used in combination)

1 ml of a reaction mixture comprising 80 μl of the concentrated cells obtained in Example 22, 300 mM potassium phosphate buffer solution, 10 mg of racemic 1-phenyl-1,2-ethanediol, 40 mg of glucose, 1 mg of NAD+, 1 mg of NADP+, and 300 U of the water-producing NADH oxidase derived from *Streptococcus mutans* obtained in Example 9, was shaken in a test tube at 20°C, and while adjusting to pH 7 with 5 M sodium hydroxide, they were reacted for 27 hours. After completion of the reaction, an analysis was performed by the same method as described in Example 23 (content

analysis: column temperature 150°C, optical purity analysis: column temperature 80°C), and the percent recovery and the optical purity were calculated. As a result, (S)-1-phenyl-1,2-ethanediol having an optical purity of 93.3% e.e. was produced at a percent recovery of 100%.

(Example 29) Synthesis of (S)-4-phenyl-1,2-butanediol (a 3-enzyme co-producing bacterium and an oxidized form coenzyme regeneration system using a water-producing NADH oxidase were used in combination)

1 ml of a reaction mixture comprising 80 μl of the concentrated cells obtained in Example 22, 300 mM potassium phosphate buffer solution, 10 mg of racemic 4-phenyl-1,2-butanediol, 40 mg of glucose, 1 mg of NAD+, 1 mg of NADP+, and 300 U of the water-producing NADH oxidase derived from *Streptococcus mutans* obtained in Example 9, was shaken in a test tube at 20°C, and while adjusting to pH 7 with 5 M sodium hydroxide, they were reacted for 27 hours. After completion of the reaction, an analysis was performed by the same method as described in Example 23 (content analysis: column temperature 180°C, optical purity analysis: column temperature 115°C), and the percent recovery and the optical purity were calculated. As a result, (S)-4-phenyl-1,2-pentanediol having an optical purity of 100% e.e. was produced at a percent recovery of 97%.

(Example 30) Synthesis of (S)-3-chloro-1,2-propanediol (an oxidized form coenzyme regeneration system using an NADH oxidase and a reduced form coenzyme regeneration system using an NADP+-specific glucose dehydrogenase were used in combination)

Each of *E. coli* HB101 (pTSOB) FERM BP-10461 and *E. coli* HB101 (pNTGDH-J3687) FERM P-20374 (see Example 15) was cultured using a 2×YT medium (Bacto Trypton 1.6%, Bacto Yeast Extract 1.0%, NaCl 0.5%, pH 7.0) containing 100 μg/ml ampicillin, and cells were harvested, after which they were suspended in 100 mM phosphate buffer solution (pH 7.0). Thereafter, the cells were homogenized by sonication using a UH-50 model ultrasonic homogenizer (manufactured by SMT Company), and the homogenized residue was removed by centrifugation, to prepare the NAD+-specific dehydrogenase derived from *Ochrobactrum* sp. and the NADP+-specific glucose dehydrogenase derived from *Cryptococcus uniguttulatus.*

1 ml of a reaction mixture comprising 300 mM potassium phosphate buffer solution, 70 mg of racemic 3-chloro-1,2-propanediol, 114 mg of glucose, 1 mg of NAD+, 1 mg of NADP+, 7 U (as 3-chloro-1,2-propanediol oxidation activity) of the foregoing NAD+-specific dehydrogenase (oxidizing enzyme) derived from *Ochrobactrum* sp., 600 U (as 3-chloro-1-hydroxyacetone reduction activity) of the NADPH-specific dehydrogenase (reducing enzyme) derived from *Rhodotorula glutinis* obtained in Example 14, 300 U of the water-producing NADH oxidase derived from *Streptococcus mutans* obtained in Example 9 (an enzyme for regenerating an oxidized form coenzyme), and 25 U of the foregoing NADP+-specific glucose dehydrogenase derived from *Cryptococcus uniguttulatus* (an enzyme for regenerating a reduced form coenzyme), was shaken in a test tube at 20°C, and while adjusting to pH 7 with 5 M sodium hydroxide, they were reacted for 29 hours. After completion of the reaction, an analysis was performed by the method described in Example 1, and the percent recovery and the optical purity were calculated. As a result, (S)-3-chlorol,2-propanediol having an optical purity of 99.2% e.e. was produced at a percent recovery of 92%.

(Example 31) Synthesis of (R)-2-pentanol (an oxidized form coenzyme regeneration system using an NADH oxidase and a reduced form coenzyme regeneration system using an NADP+-specific glucose dehydrogenase were used in combination)

1 ml of a reaction mixture comprising 300 mM potassium phosphate buffer solution, 10 mg of racemic 2-pentanol, 40 mg of glucose, 1 mg of NAD+, 1 mg of NADP+, 7 U (as 3-chloro-1,2-propanediol oxidation activity) of the NAD+-specific dehydrogenase (oxidizing enzyme) derived from *Ochrobactrum* sp. obtained in Example 30, 100 U (as 3-chloro-1-hydroxyacetone reduction activity) of the NADPH-specific dehydrogenase (reducing enzyme) derived from *Candida magnoriae* obtained in Example 11, 300 U of the water-producing NADH oxidase derived from *Streptococcus mutans* obtained in Example 9 (an enzyme for regenerating an oxidized form coenzyme), and 30 U of the NADP+-specific glucose dehydrogenase derived from *Cryptococcus* uniguttulatus obtained in Example 30 (an enzyme for regenerating a reduced form coenzyme), was shaken in a test tube at 20°C, and while adjusting to pH 7 with 5 M sodium hydroxide, they were reacted for 2 hours. After completion of the reaction, the reaction product was analyzed by the same method as described in Example 23 (content analysis, optical purity analysis: column temperature 30°C), and the percent recovery and the optical purity were calculated. As a result, (R)-2-pentanol having an optical purity of 100% e.e. was produced at a percent recovery of 100%.

(Example 32) Synthesis of (R)-1-phenylethanol (an oxidized form coenzyme regeneration system using an NADH oxidase and a reduced form coenzyme regeneration system using an NADP+-specific glucose dehydrogenase were used in combination)

1 ml of a reaction mixture comprising 300 mM potassium phosphate buffer solution, 10 mg of racemic 1-phenylethanol, 40 mg of glucose, 1 mg of NAD+, 1 mg of NADP+, 7 U (as 3-chloro-1,2-propanediol oxidation activity) of the NAD+-specific dehydrogenase (oxidizing enzyme) derived from *Ochrobactrum* sp. obtained in Example 30, 100 U (as 3-chloro-1-hydroxyacetone reduction activity) of the NADPH-specific dehydrogenase (reducing enzyme) derived from *Candida ma gnoriae* obtained in Example 11, 300 U of the water-producing NADH oxidase derived from *Streptococcus mutans* obtained in Example 9 (an enzyme for regenerating an oxidized form coenzyme), and 30 U of the NADP+-specific glucose dehydrogenase derived from *Cryptococcus uniguttulatus* obtained in Example 30 (an enzyme for regenerating a reduced form coenzyme), was shaken in a test tube at 20°C, and while adjusting to pH 7 with 5 M sodium hydroxide, they were reacted for 2 hours. After completion of the reaction, an analysis was performed by the method described in Example 14, and the percent recovery and the optical purity were calculated. As a result, (R)-1-phenylethanol having an optical purity of 99.7% e.e. was produced at a percent recovery of 98%.

(Example 33) Synthesis of (R)-1,3-butanediol (an oxidized form coenzyme regeneration system using an NADH oxidase and a reduced form coenzyme regeneration system using an NADP+-specific glucose dehydrogenase were used in combination)

1 ml of a reaction mixture comprising 300 mM potassium phosphate buffer solution, 10 mg of racemic 1,3-butanediol, 40 mg of glucose, 1 mg of NAD+, 1 mg of NADP+, 7 U (as 3-chloro-1,2-propanediol oxidation activity) of the NAD+-specific dehydrogenase (oxidizing enzyme) derived from *Ochrobactrum* sp. obtained in Example 30, 100 U (as 3-chloro-1-hydroxyacetone reduction activity) of the NADPH-specific dehydrogenase (reducing enzyme) derived from *Candida magnoriae* obtained in Example 11, 300 U of the water-producing NADH oxidase derived from *Streptococcus mutans* obtained in Example 9 (an enzyme for regenerating an oxidized form coenzyme), and 30 U of the NADP+-specific glucose dehydrogenase derived from *Cryptococcus uniguttulatus* obtained in Example 30 (an enzyme for regenerating a reduced form coenzyme), was shaken in a test tube at 20°C, and while adjusting to pH 7 with 5 M sodium hydroxide, they were reacted for 8 hours. After completion of the reaction, an analysis was performed by the method described in Example 4, and the percent recovery and the optical purity were calculated. As a result, (R)-1,3-butanediol having an optical purity of 76.8% e.e. was produced at a percent recovery of 70%.

(Example 34) Preparation of a recombinant vector comprising the dehydrogenase gene derived from *Ochrobactrum* sp., the dehydrogenase gene derived from *Rhodotorula glutinis,* and the glucose dehydrogenase gene derived from *Cryptococcus uniguttulatus,* and a recombinant *Escherichia coli* (3-enzyme co-producing bacterium)

To co-produce three enzymes, the NAD+-specific dehydrogenase (oxidizing enzyme) derived from *Ochrobactrum* sp., the NADPH-specific dehydrogenase (reducing enzyme) derived from *Rhodotorula glutinis,* and the NADP+-specific glucose dehydrogenase derived from *Cryptococcus uniguttulatus* (an enzyme for regenerating a reduced form coenzyme) in *Escherichia coli,* a recombinant vector for use in transformation was prepared.

First, a double-stranded DNA having a BamHI site and the SD sequence added to the initiation site of the structural gene of the dehydrogenase derived from *Rhodotorula glutinis,* and also having a new stop codon and an XbaI site added just after the original stop codon, was acquired by the method described below. Using sequence primer-21 (aagccggatcctaaggaggttaacaatgcccgcagcaaagactta: SEQ ID NO:24) and primer-22 (ttgcgtctagattactaccacggcacggtcttgc: SEQ ID NO:25), with the plasmid pNTRG comprising the dehydrogenase gene derived from *Rhodotorula glutinis* (see WO03/093477) as the template, PCR was performed to yield a double-stranded DNA. This double-stranded DNA was digested with BamHI and XbaI and inserted to the BamHI and XbaI sites of the plasmid pTSOB comprising the dehydrogenase gene derived from *Ochrobactrum* sp. (see Figure 2), whereby the recombinant vector pTSOBRG was obtained.

Next, a double-stranded DNA having an XbaI site and the SD sequence added to the initiation site of the structural gene of the NADP+-specific glucose dehydrogenase derived from *Cryptococcus uniguttulatus,* and also having an SphI site added just after the stop codon, was acquired by the method described below. Using the foregoing primer-19 (SEQ ID NO:22) and primer-23 (ttgcggcatgcttactgcaaaccagccgtgt:SEQ ID NO:26), with the plasmid pNTGDH-J3687 comprising the NADP+-specific glucose dehydrogenase gene derived from *Cryptococcus uniguttulatus* (see Example 15) as the template, PCR was performed to yield a double-stranded DNA. This double-stranded DNA was digested with XbaI and SphI and inserted to the XbaI and SphI sites of the above-described pTSOBRG, whereby the recombinant vector pTSOBRGGP was obtained. Using this recombinant vector pTSOBRGGP, *E. coli* HB101 (manufactured by Takara Shuzo Co., Ltd.) was transformed, to yield the 3-enzyme co-producing bacterium *E. coli* HB101 (pTSOBRGGP).

(Example 35) Synthesis of (S)-3-chloro-1,2-propanediol (a 3-enzyme co-producing bacterium and a water-producing NADH oxidase were used in combination)

The 3-enzyme co-producing bacterium *E. coli* HB101 (pTSOBRGGP) obtained in Example 34 was inoculated to 50 ml of 2×YT medium (Bacto Trypton 1.6%, Bacto Yeast Extract 1.0%, NaCl 0.5%, pH 7.0) sterilized in a 500 ml capacity Sakaguchi flask, and subjected to shaking culture at 30°C for 36 hours. 50 ml of the above-described culture broth was centrifuged, and cells were harvested and suspended in 4 ml of 100 mM phosphate buffer solution (pH 7.0) (12.5 fold concentrated cells).

1 ml of a reaction mixture comprising 80 μl of the above-described concentrated cells, 300 mM potassium phosphate buffer solution, 70 mg of racemic 3-chloro-1,2-propanediol, 1 mg of NAD+, 1 mg of NADP+, and 300 U of the water-producing NADH oxidase derived from *Streptococcus mutans* obtained in Example 9, was shaken in a test tube at 20°C, and while adjusting to pH 7 with 5 M sodium hydroxide, they were reacted for 27 hours. After completion of the reaction, an analysis was performed by the method described in Example 1, and the percent recovery and the optical purity were calculated. As a result, (S)-3-chloro-1,2-propanediol having an optical purity of 99.8% e.e. was produced at a percent recovery of 100%.

(Example 36) Preparation of a recombinant vector comprising the glycerol dehydrogenase gene derived from *Cellulomonas* sp., the NADH oxidase gene derived from *Streptococcus mutans,* the dehydrogenase gene derived from *Candida magnoriae,* and the glucose dehydrogenase gene derived from *Cryptococcus uniguttulatus,* and a recombinant *Escherichia coli* (4-enzyme co-producing bacterium)

To co-produce 4 enzymes, the NAD+-specific glycerol dehydrogenase derived from *Cellulomonas* sp. (oxidizing enzyme), the water-producing NADH oxidase derived from *Streptococcus mutans* (an enzyme for regenerating an oxidized form coenzyme), the NADPH-specific dehydrogenase derived from *Candida magnoriae* (reducing enzyme), and the NADP+-specific glucose dehydrogenase derived from *Cryptococcus uniguttulatus* (an enzyme for regenerating a reduced form coenzyme) in *Escherichia coli,* a recombinant vector for transformation was prepared.

First, a double-stranded DNA having a HindIII site and the SD sequence added to the initiation site of the structural gene of the water-producing NADH oxidase, and also having a new stop codon and a HindIII site added just after the original stop codon, was acquired by the method described below. Using a combination of synthetic primers, primer-24 (aagccaagctttaaggaggttaacaatgagtaaaatcgttattgt: SEQ ID NO:27) and primer-25 (ttgccaaaataagtttctcatagcttt: SEQ ID NO:28), and a combination of primer-26 (aaagctatgagaaacttattttggcaa: SEQ ID NO:29) and primer-27 (ttgcgaagcttttatcatttagcttttaatgctg: SEQ ID NO:30), with the plasmid pNTNX comprising the water-producing NADH oxidase gene as the template, PCR was performed to synthesize double-stranded DNAs (c) and (d), respectively. Furthermore, using the foregoing primer-16 (SEQ ID NO:19) and the foregoing primer-19 (SEQ ID NO:22), with a mixture of the double-stranded DNAs (c) and (d) obtained above as the template, PCR was performed to yield a double-stranded DNA. The DNA fragment obtained was digested with HindIII and inserted to the HindIII site of the plasmid pNTS1CSGP described in Example 21, whereby the recombinant vector pNTS1CSGPNX was obtained. Using this recombinant vector pNTS1CSGPNX, *E. coli* HB101 was transformed, to yield the 4-enzyme co-producing bacterium E. coli HB101 (pNTS1CSGPNX).

(Example 37) Synthesis of (R)-3-chloro-1,2-propanediol (a 4-enzyme co-producing bacterium used)

The 4-enzyme co-producing bacterium E. *coli* HB101 (pNTS1CSGPNX) obtained in Example 36 was inoculated to 50 ml of 2×YT medium (Bacto Trypton 1.6%, Bacto Yeast Extract 1.0%, NaCl 0.5%, pH 7.0) sterilized in a 500 ml capacity Sakaguchi flask, and subjected to shaking culture at 30°C for 36 hours. 50 ml of the above-described culture broth of each of E. *coli* HB101 (pTSCSNX) and *E. coli* HB101 (pNTS1G-J) was centrifuged, and cells were harvested and suspended in 4 ml of 100 mM phosphate buffer solution (pH 7.0).

1 ml of a reaction mixture comprising 120 μl of the above-described suspension of E. *coli* HB101 (pNTS1CSGPNX) cells, 300 mM potassium phosphate buffer solution, 70 mg of racemic 3-chloro-1,2-propanediol, 114 mg of glucose, 1 mg of NAD+, and 1 mg of NADP+, was shaken in a test tube at 20°C, and while adjusting to pH 7 with 5 M sodium hydroxide, they were reacted for 20 hours. After completion of the reaction, an analysis was performed by the method described in Example 1, and the percent recovery and the optical purity were calculated. As a result, (R)-3-chloro-1,2-propanediol having an optical purity of 100% e.e. was produced at a percent recovery of 95%.

(Comparative Example 1) Synthesis of (R)-3-chloro-1,2-propanediol (oxidative reaction)

1 ml of the cell-free extract of E. *coli* HB101 (pTSCS) obtained in Example 2, 10 mg of racemic 3-chloro-1,2-propanediol, and 35 mg of NAD+ were added to a stoppered test tube, and while adjusting to pH 8.0 with 2 M sodium

hydroxide aqueous solution, this mixture was stirred at 30°C for 92 hours. After completion of the reaction, an analysis was performed by the same method as Example 1; as a result, the percent recovery was 60%, and the optical purity was 48.6% e.e.(R).

(Comparative Example 2) Synthesis of (R)-3-chloro-1,2-propanediol utilizing the NAD regeneration capability of *Escherichia coli* for regenerating an oxidized form coenzyme (oxidative reaction)

1 ml of the culture broth of E. *coli* HB101 (pTSCS) obtained in Example 1 and 10 mg of racemic 3-chloro-1,2-propanediol were added to a stoppered test tube, and while adjusting to pH 8.0 with 2 M sodium hydroxide aqueous solution, this mixture was stirred at 30°C for 92 hours. After completion of the reaction, an analysis was performed by the same method as Comparative Example 1; as a result, the percent recovery was 41%, and the optical purity was 99.8% e.e.(R).

(Comparative Example 3) Synthesis of (R)-3-chloro-1,2-propanediol utilizing an amino acid dehydrogenase for regenerating an oxidized form coenzyme (oxidative reaction)

1 ml of the cell-free extract of E. *coli* HB101 obtained in Example 2 (pTSCS), 10 mg of racemic 3-chloro-1,2-propanediol, 1.0 mg of NAD+, 10 U of leucine dehydrogenase (manufactured by SIGMA Company), 15.2 mg of sodium α-ketoisovalerate, and 5.3 mg of ammonium chloride were added to a stoppered test tube, and while adjusting to pH 8.0 with 2 M sodium hydroxide aqueous solution, this mixture was stirred at 30°C for 92 hours. After completion of the reaction, an analysis was performed by the same method as Comparative Example 1; as a result, the percent recovery was 37%, and the optical purity was 97.1% e.e.(R).

(Comparative Example 4) Synthesis of (R)-3-chloro-1,2-propanediol utilizing an NADH oxidase for regenerating an oxidized form coenzyme (oxidative reaction)

1 ml of the cell-free extract of E. *coli* HB101 (pTSCS) obtained in Example 2, 10 mg of racemic 3-chloro-1,2-propanediol, 1.0 mg of NAD+, 1 U of hydrogen peroxide-producing type NAD H oxidase (manufactured by SIGMA Company), and 20 U of catalase (manufactured by SIGMA Company) were added to a stoppered test tube, and while adjusting to pH 8.0 with 2 M sodium hydroxide aqueous solution, this mixture was stirred at 30°C for 92 hours. After completion of the reaction, an analysis was performed by the same method as Comparative Example 1; as a result, the percent recovery was 41%, and the optical purity was 64.9% e.e.(R).

## Claims

1. A method of producing an optically active secondary alcohol by converting an enantiomer mixture of secondary alcohol into an optically active secondary alcohol consisting of a substantially single enantiomer, comprising performing the converting reaction in the co-presence of an oxidizing enzyme source having the following property (1) and a reducing enzyme source having the following property (2):

   (1) the oxidizing enzyme source exhibits specificity for one of the oxidized form coenzymes NAD+ or NADP+,

and has an activity to selectively oxidize one enantiomer of the S form or R form of secondary alcohol to produce a corresponding ketone compound,

(2) the reducing enzyme source exhibits specificity for one of the reduced form coenzymes NADPH or NADH (here, if the oxidizing enzyme source is specific for NAD+, the reducing enzyme source is specific for NADPH; if the oxidizing enzyme source is specific for NADP+, the reducing enzyme source is specific for NADH), has the reverse enantio-selectivity to that of the oxidizing enzyme source, and has an activity to reduce the foregoing ketone compound to produce the S form (or R form) of secondary alcohol.

**2.** The production method of claim 1, wherein the oxidizing enzyme source exhibits specificity for the oxidized form coenzyme NAD+, and the reducing enzyme source exhibits specificity for the reduced form coenzyme NADPH.

**3.** The production method of claim 1 or 2, wherein the converting reaction is performed in combination with an oxidized form coenzyme regeneration system and/or a reduced form coenzyme regeneration system.

**4.** The production method of claim 3, comprising using a microbial cell that produces the oxidizing enzyme as the above-described oxidizing enzyme source, and utilizing the NAD+ or NADP+ regeneration capability in a microbial cell for regenerating the above-described oxidized form coenzyme.

**5.** The production method of claim 4, wherein the microorganism that produces the above-described oxidizing enzyme is a recombinant *Escherichia coli.*

**6.** The production method of claim 3, comprising using NADH oxidase, NADPH dehydrogenase, or amino acid dehydrogenase for regenerating the above-described oxidized form coenzyme.

**7.** The production method of claim 6, wherein the above-described NADH oxidase is a water-producing NADH oxidase.

**8.** The production method of claim 7, wherein the water-producing NADH oxidase is an enzyme derived from a microorganism belonging to the genus *Streptococcus.*

**9.** The production method of claim 8, wherein the microorganism of the genus *Streptococcus* is *Streptococcus mutans.*

**10.** The production method of claim 9, wherein the *Streptococcus mutans* is *Streptococcus mutans* NCIB11723.

**11.** The production method of claim 3, comprising using glucose dehydrogenase, formic acid dehydrogenase or glucose 6-phosphate dehydrogenase for regenerating the above-described reduced form coenzyme.

**12.** The production method of claim 11, wherein the above-described glucose dehydrogenase is NADP+-specific glucose dehydrogenase.

**13.** The production method of claim 12, wherein the NADP+-specific glucose dehydrogenase is an enzyme derived from a microorganism belonging to the genus *Cryptococcus.*

**14.** The production method of claim 14, wherein the microorganism of the genus *Cryptococcus* is *Cryptococcus uniguttulatus.*

**15.** The production method of claim 3, using a recombinant microbial cell prepared by expressing the following enzymes (A) and (B) in the same host cell as the oxidizing enzyme source:

(A) an enzyme that exhibits specificity for the oxidized form coenzyme NAD+, and has an activity to selectively oxidize one enantiomer of the S form or R form of secondary alcohol to produce a corresponding ketone compound,

(B) an enzyme having the capability of regenerating the oxidized form coenzyme NAD+.

**16.** The production method of claim 15, wherein the enzyme having the capability of regenerating the oxidized form coenzyme NAD+ is an NADH oxidase.

**17.** The production method of claim 16, wherein the NADH oxidase is a water-producing NADH oxidase.

**18.** The production method of claim 3, wherein a recombinant microbial cell prepared by expressing the following enzymes (C) and (D) in the same host cell is used as the reducing enzyme source:

(C) an enzyme that exhibits specificity for the reduced form coenzyme NADPH, and stereo-selectively reduces a ketone compound to produce the S form or R form of secondary alcohol,
(D) an enzyme having the capability of regenerating the reduced form coenzyme NADPH.

**19.** The production method of claim 18, wherein the enzyme having the capability of regenerating the reduced form coenzyme NADPH is a glucose dehydrogenase.

**20.** The production method of claim 19, wherein the glucose dehydrogenase is an NADP+-specific glucose dehydrogenase.

**21.** The production method of claim 3, wherein a recombinant microbial cell prepared by expressing the following enzymes (A) and (C) in the same host cell is used as the oxidizing enzyme source and reducing enzyme source:

(A) an enzyme that exhibits specificity for the oxidized form coenzyme NAD+, and has an activity to selectively oxidize one enantiomer of the S form or R form of secondary alcohol to produce a corresponding ketone compound,
(C) an enzyme that exhibits specificity for the reduced form coenzyme NADPH, and stereo-selectively reduces a ketone compound to produce the S form or R form of secondary alcohol.

**22.** The production method of claim 21, using a recombinant microbial cell prepared by expressing, in addition to the foregoing enzymes (A) and (C), the following enzyme (B) in the same host cell:
(B) an enzyme having the capability of regenerating the oxidized form coenzyme NAD+.

**23.** The production method of claim 21, using a recombinant microbial cell prepared by expressing, in addition to the foregoing enzymes (A) and (C), the following enzyme (D) in the same host cell:

(D) an enzyme having the capability of regenerating the reduced form coenzyme NADPH.

**24.** The production method of claim 21, using a recombinant microbial cell prepared by expressing, in addition to the foregoing enzymes (A) and (C), the following enzymes (B) and (D) in the same host cell:

(B) an enzyme having the capability of regenerating the oxidized form coenzyme NAD+,
(D) an enzyme having the capability of regenerating the reduced form coenzyme NADPH.

**25.** A recombinant microorganism prepared by expressing the following enzymes (A) and (B) in the same host cell:

(A) an enzyme that exhibits specificity for the oxidized form coenzyme NAD+, and has an activity to selectively oxidize one enantiomer of the S form or R form of secondary alcohol to produce a corresponding ketone compound,
(B) an enzyme having the capability of regenerating the oxidized form coenzyme NAD+.

**26.** The recombinant microorganism of claim 25, wherein the enzyme having the capability of regenerating the oxidized form coenzyme NAD+ is an NADH oxidase.

**27.** The recombinant microorganism of claim 25, wherein the NADH oxidase is a water-producing NADH oxidase.

**28.** A recombinant microorganism prepared by expressing the following enzymes (C) and (D) in the same host cell:

(C) an enzyme that exhibits specificity for the reduced form coenzyme NADPH, and stereo-selectively reduces a ketone compound to produce the S form or R form of secondary alcohol,
(D) an enzyme having the capability of regenerating the reduced form coenzyme NADPH.

**29.** The recombinant microorganism of claim 28, wherein the enzyme having the capability of regenerating the reduced form coenzyme NADPH is a glucose dehydrogenase.

**30.** The recombinant microorganism of claim 29, wherein the glucose dehydrogenase is an NADP+-specific glucose dehydrogenase.

**31.** A recombinant microorganism prepared by expressing the following enzyme (A) or (C) in the same host cell:

(A) an enzyme that exhibits specificity for the oxidized form coenzyme NAD+, and has an activity to selectively oxidize one enantiomer of the S form or R form of secondary alcohol to produce a corresponding ketone compound,
(C) an enzyme that exhibits specificity for the reduced form coenzyme NADPH, has the reverse enantio-selectivity to that of (A), and stereo-selectively reduces the foregoing ketone compound to produce the S form or R form of secondary alcohol.

**32.** The recombinant microorganism of claim 31, expressing, in addition to the foregoing enzymes (A) and (C), the following enzyme (B) in the same host cell:
(B) an enzyme having the capability of regenerating the oxidized form coenzyme NAD+.

**33.** The recombinant microorganism of claim 31, expressing, in addition to the foregoing enzymes (A) and (C), the following enzyme (D) in the same host cell:

(D) an enzyme having the capability of regenerating the reduced form coenzyme NADPH.

**34.** The recombinant microorganism of claim 31, expressing, in addition to the foregoing enzymes (A) and (C), the following enzymes (B) and (D) in the same host cell:

(B) an enzyme having the capability of regenerating the oxidized form coenzyme NAD+,
(D) an enzyme having the capability of regenerating the reduced form coenzyme NADPH.

FIG. 1

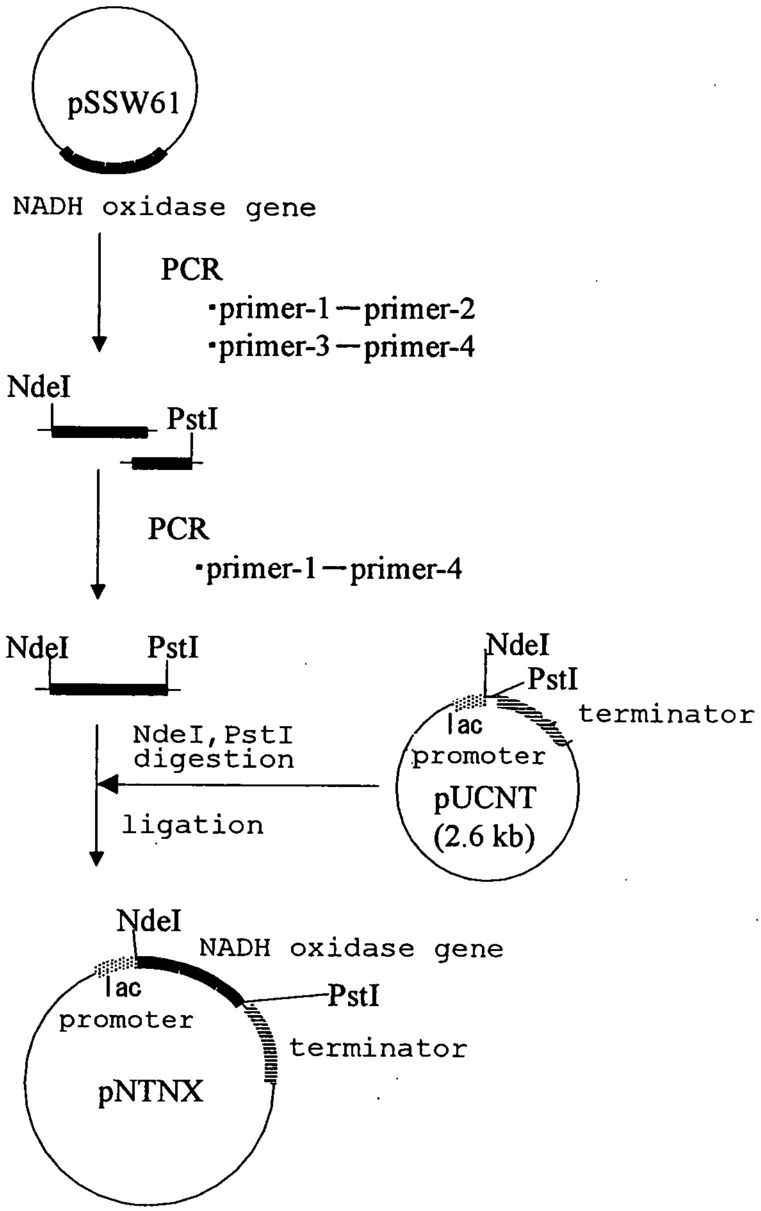
pSSW61
NADH oxidase gene
PCR
·primer-1—primer-2
·primer-3—primer-4
NdeI
PstI
PCR
·primer-1—primer-4
NdeI
PstI
NdeI
PstI
terminator
lac
promoter
pUCNT
(2.6 kb)
NdeI, PstI
digestion
ligation
NdeI
NADH oxidase gene
lac
promoter
PstI
terminator
pNTNX

FIG. 2

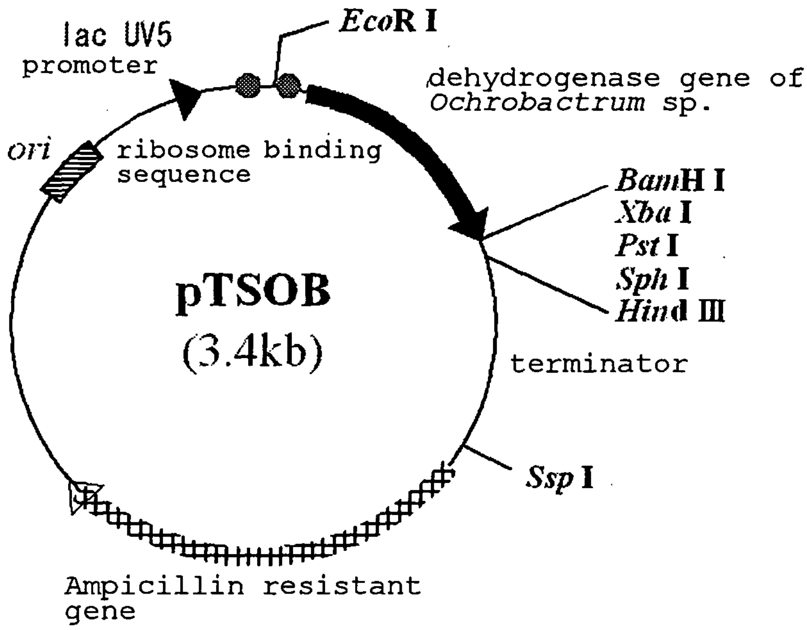
lac UV5
promoter
EcoR I
dehydrogenase gene of
Ochrobactrum sp.
ori
ribosome binding
sequence
BamH I
Xba I
Pst I
Sph I
Hind III
pTSOB
(3.4kb)
terminator
Ssp I
Ampicillin resistant
gene

**INTERNATIONAL SEARCH REPORT**

International application No.
PCT/JP2006/303369

A. CLASSIFICATION OF SUBJECT MATTER
*C12P41/00*(2006.01), *C12N15/53*(2006.01)

According to International Patent Classification (IPC) or to both national classification and IPC

B. FIELDS SEARCHED

Minimum documentation searched (classification system followed by classification symbols)
C12P41/00, C12N15/00-15/90

Documentation searched other than minimum documentation to the extent that such documents are included in the fields searched

Electronic data base consulted during the international search (name of data base and, where practicable, search terms used)
BIOSIS/WPI(DIALOG)

C. DOCUMENTS CONSIDERED TO BE RELEVANT

| Category* | Citation of document, with indication, where appropriate, of the relevant passages | Relevant to claim No. |
|---|---|---|
| Y<br>A | JP 2002-330781 A (Degssa AG.), 19 November, 2002 (19.11.02), & EP 1176203 A1 & US 2003/0054520 A1 | 1,2<br>3-24,32-34 |
| Y<br>A | JP 2004-105089 A (Asahi Kasei Fama Kabushiki Kaisha), 08 April, 2004 (08.04.04), (Family: none) | 1,2<br>3-24,32-34 |
| Y<br>A | JP 2002-345479 A (Daicel Chemical Industries, Ltd.), 03 December, 2002 (03.12.02), & EP 1262550 A3 & US 2003/0032153 A1 | 1,2<br>3-24,32-34 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 May, 2006 (17.05.06) | 23 May, 2006 (23.05.06) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2006/303369

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet.

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
Claims 1-24 and 32-34

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2005)

**INTERNATIONAL SEARCH REPORT**

International application No.
PCT/JP2006/303369

Continuation of Box No.III of continuation of first sheet(2)

<<Documents>>

1. JP 2002-330781 A
2. JP 2004-105089 A

The subject matter of claims 1-24 and 32-34 (hereinafter, referred to as "invention group 1") is a technique in which an oxidase source showing specificity for one of coenzymes NAD+ and NADP+ and having the ability to selectively oxidize one of the (S) and (R) enantiomers of a secondary alcohol to yield the corresponding ketone compound is caused to coexist with a reductase source which shows specificity for the coenzyme different from that for which the oxidase source shows specificity and which reduces a ketone compound to selectively yield the secondary-alcohol enantiomer other than that.

The subject matter of claims 25-27 (hereinafter, referred to as "invention group 2") is a technique in which an enzyme showing specificity for coenzyme NAD+ and having the ability to selectively oxidize one of the (S) and (R) enantiomers of a secondary alcohol to yield the corresponding ketone compound and an enzyme having the ability to regenerate NAD+ are expressed in the same host cell.

The subject matter of claims 28-30 (hereinafter, referred to as "invention group 3") is a technique in which an enzyme showing specificity for NADPH and stereoselectively reducing a ketone compound to yield the (S) or (R) enantiomer of a secondary alcohol and an enzyme having the ability to regenerate NADPH are expressed in the same host cell.

That part of claim 31 which relates to (A) (hereinafter, referred to as "invention group 4") is a technique in which an enzyme showing specificity for coenzyme NAD+ and having the ability to selectively oxidize one of the (S) and (R) enantiomers of a secondary alcohol to yield the corresponding ketone compound is expressed in a host cell.

That part of claim 31 which relates to (C) (hereinafter, referred to as "invention group 5") is a technique in which an enzyme showing specificity for NADPH and stereoselectively reducing a ketone compound to yield the (S) or (R) enantiomer of a secondary alcohol is expressed in a host cell.

A matter common between invention groups 1 and 2 is an enzyme which shows specificity for coenzyme NAD+ and has the ability to selectively oxidize one of the (S) and (R) enantiomers of a secondary alcohol to yield the corresponding ketone compound.

However, an enzyme having those properties is described in the document 1, claims 1 and 13 and paragraph No. [0021]. Consequently, the two invention groups are not considered to have a common technical feature which contributes to the prior art. They are not considered to be so linked as to form a single general inventive concept.

A matter common between invention groups 1 and 3 is an enzyme which shows specificity for NADPH and stereoselectively reduces a ketone compound to yield the (S) or (R) enantiomer of a secondary alcohol.

However, an enzyme catalyzing asymmetric carbonyl reduction having those properties is described in the document 2, claim 1. Consequently, the two invention groups are not considered to have a common technical feature which contributes to the prior art. They are not considered to be so linked as to form a single general inventive concept.

(continued to next page)

INTERNATIONAL SEARCH REPORT

International application No.
PCT/JP2006/303369

A matter common between invention groups 1 and 4 is an enzyme which has the ability to selectively oxidize one of the (S) and (R) enantiomers of a secondary alcohol to yield the corresponding ketone compound. However, an enzyme having those properties is described in the document 1 as stated above. Consequently, the two invention groups are not considered to have a common technical feature which contributes to the prior art. They are not considered to be so linked as to form a single general inventive concept.

A matter common between invention groups 1 and 5 is an enzyme which shows specificity for NADPH and stereoselectively reduces a ketone compound to yield the (S) or (R) enantiomer of a secondary alcohol. However, an enzyme having those properties is described in the document 2 as stated above. Consequently, the two invention groups are not considered to have a common technical feature which contributes to the prior art. They are not considered to be so linked as to form a single general inventive concept.

Even when other combinations of the invention groups 1-5 are compared, none of the combinations is considered to have a common technical feature which contributes to the prior art. They are hence not considered to be so linked as to form a single general inventive concept.

Therefore, this application involves five inventions in total, i.e., the subject matter of claims 1-24 and 32-34, the subject matter of claims 25-27, the subject matter of claims 28-30, that part of claim 31 which relates to (A), and that part of claim 31 which relates to (C).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 63251098 A
- JP 2002345479 A
- JP 8196281 A
- WO 05123921 A
- WO 01005996 A
- WO 98035025 A
- WO 03093477 A
- WO 9835025 A
- WO 9403613 A

### Non-patent literature cited in the description

- *Agric. Biol. Chem,* 1990, vol. 54 (7), 1819-1827
- *Organic Process Research & Development,* 2004, vol. 8 (2), 246-251
- **HIGASHI ; TSUKUBA ; IBARAKI.** International Patent Organism Depositary, National Institute of Advanced Industrial Science and Technology. vol. 6, 1-1
- *Biosci. Biotech. Biochem.,* 1996, vol. 60 (1), 39-43
- **HIGASHI ; TSUKUBA ; IBARAKI.** International Patent Organism Depositary, National Institute of Advanced Industrial Science and Technology. 21 January 2005, vol. 6, 1-1

SEQUENCE LISTING

<110> Kaneka Corporation

<120> PROCESS FOR PRODUCING OPTICALLY ACTIVE SECONDARY ALCOHOL

<130> B050071W001-

<150> JP2005-050488
<151> 2005-02-25

<160> 30

<170> PatentIn version 3.1

<210> 1
<211> 31
<212> DNA
<213> Artificial

<220>
<223> primer-1

<400> 1
gaggatttgc atatgagtaa aatcgttatt g 31

<210> 2
<211> 31
<212> DNA
<213> Artificial

<220>
<223> primer-2

<400> 2
atgaaaacat gtgaattccc attgacatat c 31

<210> 3
<211> 31
<212> DNA
<213> Artificial

<220>
<223> primer-3

<400> 3
gatatgtcaa tgggaattca catgttttca t 31

<210> 4
<211> 31
<212> DNA
<213> Artificial

<220>
<223> primer-4

<400> 4
tttctgcagt tatcatttag cttttaatgc t 31

<210> 5
<211> 26
<212> DNA
<213> Artificial

<220>
<223> primer-5

<400> 5
gagaagcagc acaagatyaa rgayca 26

<210> 6

<211> 24
<212> DNA
<213> Artificial

<220>
<223> primer-6

<220>
<221> misc_feature
<222> (13)..(13)
<223> n represents a, t, g or c.

<400> 6
catgtgrgcr agngargtra aytg 24

<210> 7
<211> 742
<212> DNA
<213> Cryptococcus uniguttulatus

<400> 7
atgtttcatc gttggtgaga gcgtctcttt ggaataacgt gttgatatac ccaaagttga 60

ctgtattctc ttgccttttt caagtgtttg gagcttccgg ggatcttgca aagaagatgg 120

tgagtgtggt ttccctctgc gagatagtca tgtgttggat cgctcacgtc atcatctcgt 180

cacaacggca caacgtgcct gcccattccc tggtaaccta cagactttcc cgtccttgta 240

ccaattgttc tcccttgatc ttttgcccaa gaggaccaag attgtcggat atgctcgatc 300

caagatggac aatgaaaagt ttatggacca atatgtcggt ggactgccca aagacgccgt 360

aagtatagac ttgtggtttg cagcagatga accggagaga ccgtgtacct aattcatcgg 420

agcttttgtt acaatcaaca ggacaagaaa aaggttgaag agtttaaaaa gttggccgag 480

tacgttcagg gagcgtatga cgaggatgag gctttccagg taggttgtac atctaatcga 540

ctcatgtcct ttgaagctca cactgtgtat cctgcaacac agaaattgga aaaggatttg 600

caagagactg cagacaagga gatggatgga aaggctcatc gagtctacta cgtaagtgtt 660

tatgcatctg gacagacctg ggcaacacga ctcatgtcgc ctgtctgcaa ctttgcacac 720

acagcttgct gtgcctcctt ct 742

<210> 8
<211> 31
<212> DNA
<213> Artificial

<220>
<223> primer-7

<400> 8
agttggccga gtacgttcag ggagcgtatg a 31

<210> 9
<211> 31
<212> DNA
<213> Artificial

<220>
<223> primer-8

<400> 9
ggaaagcctc atcctcgtca tacgctccct g 31

<210> 10
<211> 23

```
<212>  DNA
<213>  Artificial

<220>
<223>  primer-9

<400>  10
cgctacgtaa cggcatgaca gtg                                              23


<210>  11
<211>  26
<212>  DNA
<213>  Artificial

<220>
<223>  primer-10

<400>  11
ggacactgac atggactgaa ggagta                                           26


<210>  12
<211>  1773
<212>  DNA
<213>  Cryptococcus uniguttulatus

<220>
<221>  CDS
<222>  (94)..(1620)
<223>

<400>  12
cttttgacta gtactaccac atcgactctt tgtcgtcgca cacgaatcag agctataccc      60

ctcgcaagaa cgatatcaaa cagacctgcc aaa atg tcg agc acc gaa ttt cag      114
                                     Met Ser Ser Thr Glu Phe Gln
                                     1               5

cac gag atc aag gac caa tgt ttc atc gtt gtg ttt gga gct tcc ggg      162
His Glu Ile Lys Asp Gln Cys Phe Ile Val Val Phe Gly Ala Ser Gly
        10                  15                  20

gat ctt gca aag aag atg act ttc ccg tcc ttg tac caa ttg ttc tcc      210
Asp Leu Ala Lys Lys Met Thr Phe Pro Ser Leu Tyr Gln Leu Phe Ser
    25                  30                  35

ctt gat ctt ttg ccc aag agg acc aag att gtc gga tat gct cga tcc      258
Leu Asp Leu Leu Pro Lys Arg Thr Lys Ile Val Gly Tyr Ala Arg Ser
40                  45                  50                  55

aag atg gac aat gaa aag ttt atg gac caa tat gtc ggt gga ctg ccc      306
Lys Met Asp Asn Glu Lys Phe Met Asp Gln Tyr Val Gly Gly Leu Pro
                60                  65                  70

aaa gac gcc gac aag aaa aag gtt gaa gag ttt aaa aag ttg gcc gag      354
Lys Asp Ala Asp Lys Lys Lys Val Glu Glu Phe Lys Lys Leu Ala Glu
            75                  80                  85

tac gtt cag gga gcg tat gac gag gat gag gct ttc cag aaa ttg gaa      402
Tyr Val Gln Gly Ala Tyr Asp Glu Asp Glu Ala Phe Gln Lys Leu Glu
        90                  95                  100

aag gat ttg caa gag act gca gac aag gag atg gat gga aag gct cat      450
Lys Asp Leu Gln Glu Thr Ala Asp Lys Glu Met Asp Gly Lys Ala His
    105                 110                 115

cga gtc tac tac ctt gct gtg cct cct tct cag ttc act tca ttg gcc      498
Arg Val Tyr Tyr Leu Ala Val Pro Pro Ser Gln Phe Thr Ser Leu Ala
120                 125                 130                 135

cac atg ttg gcc aag aac aac aaa ccc aaa gat ggc gac aag tca aag      546
His Met Leu Ala Lys Asn Asn Lys Pro Lys Asp Gly Asp Lys Ser Lys
                140                 145                 150

gtc ctc tca agc cgt ctc gtc att gag aaa cct ttt ggc aag gac tct      594
```

```
Val Leu Ser Ser Arg Leu Val Ile Glu Lys Pro Phe Gly Lys Asp Ser
            155                 160                 165

gac tct tcc ctc aag atg atg aag gac att tat gcc gcc ggc tgg aag        642
Asp Ser Ser Leu Lys Met Met Lys Asp Ile Tyr Ala Ala Gly Trp Lys
        170                 175                 180

gac gaa gaa atg tac cga gtg gac cac ttt gcc gcc gtc gag acc gtc        690
Asp Glu Glu Met Tyr Arg Val Asp His Phe Ala Ala Val Glu Thr Val
    185                 190                 195

aag caa atg ccg tac ttg atc ttt gca aac cct cac ccc atc tcg ccc        738
Lys Gln Met Pro Tyr Leu Ile Phe Ala Asn Pro His Pro Ile Ser Pro
200                 205                 210                 215

aag act ctt ttg aac aag gag aat gtc aag gct gtc atg atc gag ctc        786
Lys Thr Leu Leu Asn Lys Glu Asn Val Lys Ala Val Met Ile Glu Leu
                220                 225                 230

aag gag acg ttt ggt tgt gaa ggt cga ggt gga tac ttt gac gaa ttc        834
Lys Glu Thr Phe Gly Cys Glu Gly Arg Gly Gly Tyr Phe Asp Glu Phe
            235                 240                 245

ggt atc gtt cga gat gtc ttg caa aac cac ttt ttg caa gtc gtt agt        882
Gly Ile Val Arg Asp Val Leu Gln Asn His Phe Leu Gln Val Val Ser
        250                 255                 260

atg ctc gcc atg gac cga ccc aag tca ctt gat ggt gat cac ttg cgt        930
Met Leu Ala Met Asp Arg Pro Lys Ser Leu Asp Gly Asp His Leu Arg
    265                 270                 275

gac gaa aaa gta aag gtc ctc aaa gcg atc ccc acg ttt gac ccc aag        978
Asp Glu Lys Val Lys Val Leu Lys Ala Ile Pro Thr Phe Asp Pro Lys
280                 285                 290                 295

gac gag tcc aag tac att ctt ggt caa tac acc aag tct gaa gac ggc       1026
Asp Glu Ser Lys Tyr Ile Leu Gly Gln Tyr Thr Lys Ser Glu Asp Gly
                300                 305                 310

agc aag ccc ggg tac ctc gat gac gac agt gtc gaa aac aag gaa tca       1074
Ser Lys Pro Gly Tyr Leu Asp Asp Asp Ser Val Glu Asn Lys Glu Ser
            315                 320                 325

agg acg ggc aca ttt gcc gaa atg gtc ttg acc gtc gac aat gac cga       1122
Arg Thr Gly Thr Phe Ala Glu Met Val Leu Thr Val Asp Asn Asp Arg
        330                 335                 340

tgg aag ggc gtc ccc tgg atc ctc aag agc gcc aag gct atc gaa gag       1170
Trp Lys Gly Val Pro Trp Ile Leu Lys Ser Ala Lys Ala Ile Glu Glu
    345                 350                 355

gac att tgc cga gtc att gtc caa ctc aag cac agc gac gat gat ggc       1218
Asp Ile Cys Arg Val Ile Val Gln Leu Lys His Ser Asp Asp Asp Gly
360                 365                 370                 375

aac ttt gag tct ctc tac aag gac acc aag ccc act gcg ctc gtc ttt       1266
Asn Phe Glu Ser Leu Tyr Lys Asp Thr Lys Pro Thr Ala Leu Val Phe
                380                 385                 390

gaa ctg ttt gag ccg aaa aag gtc tac ttt agc atc aac tcg cga aag       1314
Glu Leu Phe Glu Pro Lys Lys Val Tyr Phe Ser Ile Asn Ser Arg Lys
            395                 400                 405

cca ggc ttc att ggc ggg ccc acg cga agc aag att atg ctc gac tgg       1362
Pro Gly Phe Ile Gly Gly Pro Thr Arg Ser Lys Ile Met Leu Asp Trp
        410                 415                 420

gat acg gaa gaa gcc caa aag gaa ggg aaa tcg ctt gac cct tac tct       1410
Asp Thr Glu Glu Ala Gln Lys Glu Gly Lys Ser Leu Asp Pro Tyr Ser
    425                 430                 435

gtg gtt atc gga gaa gcg ctt cgt ggt cgg gaa agc aac ttt gtg cgt       1458
Val Val Ile Gly Glu Ala Leu Arg Gly Arg Glu Ser Asn Phe Val Arg
440                 445                 450                 455

gaa gac gaa ctc gag gaa tcg tgg aga atc ttt acc ccg ttt ttg cac       1506
```

```
Glu Asp Glu Leu Glu Glu Ser Trp Arg Ile Phe Thr Pro Phe Leu His
                460                 465                 470

tat caa gag gag aac aag ggg ccc aag cct acc cct tat gcg tat ggt      1554
Tyr Gln Glu Glu Asn Lys Gly Pro Lys Pro Thr Pro Tyr Ala Tyr Gly
            475                 480                 485

tct gaa gga ccc gag cag ttc aag gag ttt gaa aag agg ttt gga tac      1602
Ser Glu Gly Pro Glu Gln Phe Lys Glu Phe Glu Lys Arg Phe Gly Tyr
        490                 495                 500

acg gct ggt ttg cag taa atgttgacgc cgtccatgtc agttgaatgt             1650
Thr Ala Gly Leu Gln
    505

atcgtcaggg tctttatgtt tgtttttttc cctgattgta tcgtaaaggc aagtagtaaa    1710

aacaaggcgt ttcatgtggt gtattcaatt tctcatctcg tcgtcaaaag aaaaaaaaaa    1770

aaa                                                                  1773


<210>  13
<211>  508
<212>  PRT
<213>  Cryptococcus uniguttulatus

<400>  13

Met Ser Ser Thr Glu Phe Gln His Glu Ile Lys Asp Gln Cys Phe Ile
1               5                   10                  15

Val Val Phe Gly Ala Ser Gly Asp Leu Ala Lys Lys Met Thr Phe Pro
            20                  25                  30

Ser Leu Tyr Gln Leu Phe Ser Leu Asp Leu Leu Pro Lys Arg Thr Lys
        35                  40                  45

Ile Val Gly Tyr Ala Arg Ser Lys Met Asp Asn Glu Lys Phe Met Asp
    50                  55                  60

Gln Tyr Val Gly Gly Leu Pro Lys Asp Ala Asp Lys Lys Lys Val Glu
65                  70                  75                  80

Glu Phe Lys Lys Leu Ala Glu Tyr Val Gln Gly Ala Tyr Asp Glu Asp
                85                  90                  95

Glu Ala Phe Gln Lys Leu Glu Lys Asp Leu Gln Glu Thr Ala Asp Lys
            100                 105                 110

Glu Met Asp Gly Lys Ala His Arg Val Tyr Tyr Leu Ala Val Pro Pro
        115                 120                 125

Ser Gln Phe Thr Ser Leu Ala His Met Leu Ala Lys Asn Asn Lys Pro
    130                 135                 140

Lys Asp Gly Asp Lys Ser Lys Val Leu Ser Ser Arg Leu Val Ile Glu
145                 150                 155                 160

Lys Pro Phe Gly Lys Asp Ser Asp Ser Ser Leu Lys Met Met Lys Asp
                165                 170                 175

Ile Tyr Ala Ala Gly Trp Lys Asp Glu Glu Met Tyr Arg Val Asp His
            180                 185                 190
```

Phe Ala Ala Val Glu Thr Val Lys Gln Met Pro Tyr Leu Ile Phe Ala
195 200 205

Asn Pro His Pro Ile Ser Pro Lys Thr Leu Leu Asn Lys Glu Asn Val
210 215 220

Lys Ala Val Met Ile Glu Leu Lys Glu Thr Phe Gly Cys Glu Gly Arg
225 230 235 240

Gly Gly Tyr Phe Asp Glu Phe Gly Ile Val Arg Asp Val Leu Gln Asn
245 250 255

His Phe Leu Gln Val Val Ser Met Leu Ala Met Asp Arg Pro Lys Ser
260 265 270

Leu Asp Gly Asp His Leu Arg Asp Glu Lys Val Lys Val Leu Lys Ala
275 280 285

Ile Pro Thr Phe Asp Pro Lys Asp Glu Ser Lys Tyr Ile Leu Gly Gln
290 295 300

Tyr Thr Lys Ser Glu Asp Gly Ser Lys Pro Gly Tyr Leu Asp Asp Asp
305 310 315 320

Ser Val Glu Asn Lys Glu Ser Arg Thr Gly Thr Phe Ala Glu Met Val
325 330 335

Leu Thr Val Asp Asn Asp Arg Trp Lys Gly Val Pro Trp Ile Leu Lys
340 345 350

Ser Ala Lys Ala Ile Glu Glu Asp Ile Cys Arg Val Ile Val Gln Leu
355 360 365

Lys His Ser Asp Asp Asp Gly Asn Phe Glu Ser Leu Tyr Lys Asp Thr
370 375 380

Lys Pro Thr Ala Leu Val Phe Glu Leu Phe Glu Pro Lys Lys Val Tyr
385 390 395 400

Phe Ser Ile Asn Ser Arg Lys Pro Gly Phe Ile Gly Gly Pro Thr Arg
405 410 415

Ser Lys Ile Met Leu Asp Trp Asp Thr Glu Glu Ala Gln Lys Glu Gly
420 425 430

Lys Ser Leu Asp Pro Tyr Ser Val Val Ile Gly Glu Ala Leu Arg Gly
435 440 445

Arg Glu Ser Asn Phe Val Arg Glu Asp Glu Leu Glu Glu Ser Trp Arg
450 455 460

Ile Phe Thr Pro Phe Leu His Tyr Gln Glu Glu Asn Lys Gly Pro Lys
465 470 475 480

Pro Thr Pro Tyr Ala Tyr Gly Ser Glu Gly Pro Glu Gln Phe Lys Glu
485 490 495

Phe Glu Lys Arg Phe Gly Tyr Thr Ala Gly Leu Gln
500 505

<210> 14
<211> 24
<212> DNA
<213> Artificial

<220>
<223> primer-11

<400> 14
acagacctgc ccatatgtcg agca 24

<210> 15
<211> 25
<212> DNA
<213> Artificial

<220>
<223> primer-12

<400> 15
ggacggcgtc tagatttact gcaaa 25

<210> 16
<211> 49
<212> DNA
<213> Artificial

<220>
<223> primer-13

<400> 16
cgcggatcct aaggaggtta acaatgagta aaatcgttat tgttggagc 49

<210> 17
<211> 27
<212> DNA
<213> Artificial

<220>
<223> primer-14

<400> 17
gcatgcctgc agttatcatt tagcttt 27

<210> 18
<211> 37
<212> DNA
<213> Artificial

<220>
<223> primer-15

<400> 18
tgtctagaca cacaggaaac acatatgtcg agcaccg 37

<210> 19
<211> 39
<212> DNA
<213> Artificial

<220>
<223> primer-16

<400> 19
agtaagctta tttactgcaa accagccgtg tatccaaac 39

<210> 20
<211> 45
<212> DNA
<213> Artificial

<220>
<223> primer-17

<400> 20
aagccgaatt ctaaggaggt taacaatgtc cgaggttccc gtccg 45

<210> 21
<211> 34
<212> DNA
<213> Artificial

<220>
<223> primer-18

<400> 21
ttgcgtctag attatcagtg ggcggtgtgc ttga 34

<210> 22
<211> 45
<212> DNA
<213> Artificial

<220>
<223> primer-19

<400> 22
aagcctctag ataaggaggt taacaatgtc gagcaccgaa tttca 45

<210> 23
<211> 31
<212> DNA
<213> Artificial

<220>
<223> primer-20

<400> 23
ttgcgaagct tttagggaag cgtgtagcca c 31

<210> 24
<211> 45
<212> DNA
<213> Artificial

<220>
<223> primer-21

<400> 24
aagccggatc ctaaggaggt taacaatgcc cgcagcaaag actta 45

<210> 25
<211> 34
<212> DNA
<213> Artificial

<220>
<223> primer-22

<400> 25
ttgcgtctag attactacca cggcacggtc ttgc 34

<210> 26
<211> 31
<212> DNA

<213> Artificial

<220>
<223> primer-23

<400> 26
ttgcggcatg cttactgcaa accagccgtg t 31

<210> 27
<211> 45
<212> DNA
<213> Artificial

<220>
<223> primer-24

<400> 27
aagccaagct ttaaggaggt taacaatgag taaaatcgtt attgt 45

<210> 28
<211> 27
<212> DNA
<213> Artificial

<220>
<223> primer-25

<400> 28
ttgccaaaat aagtttctca tagcttt 27

<210> 29
<211> 27
<212> DNA
<213> Artificial

<220>
<223> primer-26

<400> 29
aaagctatga gaaacttatt ttggcaa 27

<210> 30
<211> 34
<212> DNA
<213> Artificial

<220>
<223> primer-27

<400> 30
ttgcgaagct tttatcattt agcttttaat gctg 34